# EUROPÄISCHE PATENTSCHRIFT

(11) **EP 4 091 558 B1**
(45) Veröffentlichungstag und Bekanntmachung des Hinweises auf die Patenterteilung: **02.04.2025**
(21) Anmeldenummer: 22173693.7
(22) Anmeldetag: 17.05.2022
(51) Int. Cl.: A61B 17/29, A61B 17/00

(54) **ENDOSKOPISCHES INSTRUMENT SOWIE SCHAFT UND INSTRUMENTENEINSATZ FÜR EIN ENDOSKOPISCHES INSTRUMENT**
ENDOSCOPIC INSTRUMENT AND SHAFT AND INSTRUMENT INSERT FOR AN ENDOSCOPIC INSTRUMENT
INSTRUMENT ENDOSCOPIQUE, AINSI QUE MANCHE ET INSERT D'INSTRUMENT POUR UN INSTRUMENT ENDOSCOPIQUE

(30) Priorität: 19.05.2021 DE 102021112975
(43) Veröffentlichungstag der Anmeldung: 23.11.2022
(73) Patentinhaber: Karl Storz SE & Co. KG, 78532 Tuttlingen (DE)
(72) Erfinder: SCHNEIDER, Janosz, 78532 Tuttlingen (DE); KÄRCHER, Daniel, 78532 Tuttlingen (DE); MERZ, Robin, 78532 Tuttlingen (DE); SCHNEIDER, Sven, 78532 Tuttlingen (DE); UNGER, Tobias, 78532 Tuttlingen (DE); LÄNGLE, Dominik, 78532 Tuttlingen (DE); HOLZER, Judith, 78532 Tuttlingen (DE)

(56) Entgegenhaltungen:
- US-A1- 2002 111 534
- US-A1- 2012 259 325
- US-A1- 2017 360 462
- US-A1- 2021 275 203

## Beschreibung

Die vorliegende Erfindung betrifft ein endoskopisches Instrument, insbesondere ein endoskopisches medizinisches Instrument. Bei einem endoskopischen Eingriff wird durch eine natürliche oder eine mit Hilfe einer Inzision geschaffene künstliche Körperöffnung ein endoskopisches Instrumentarium, das insbesondere ein Endoskop und ein oder mehrere endoskopische Instrumente umfassen kann, zu einem im Körperinneren gelegenen Operationsgebiet geführt. Endoskopische Instrumente weisen hierfür einen lang erstreckten Schaft auf, an dessen distalem, d.h. benutzerfernen Ende ein Werkzeug angeordnet ist, das von einem am proximalen, d.h. benutzernahen Ende des Schafts angeordneten Handgriff über ein im Schaft angeordnetes langerstrecktes Übertragungselement betätigt werden kann. Der Schaft kann starr oder flexibel ausgebildet sein. Der Handgriff verbleibt bei einem endoskopischen Eingriff außerhalb der Körperöffnung, während der Schaft mit dem Werkzeug durch die Körperöffnung eingeführt wird.

Gemäß einer verbreiteten Bauart weisen endoskopische Instrumente einen Instrumenteneinsatz auf, der an seinem distalen Ende das Werkzeug trägt und der auch das Übertragungselement, etwa eine Zugstange, umfassen kann. Der Instrumenteneinsatz, der auch als Arbeitseinsatz bezeichnet wird, ist lösbar mit dem Schaft des Instruments verbunden. Dabei kann es vorgesehen sein, dass Instrumenteneinsätze mit unterschiedlichen Werkzeugen mit dem Schaft verbunden und von diesem wieder gelöst werden können. Insbesondere kann das Instrument zur Reinigung und/oder Sterilisation zerlegbar sein, wobei der Instrumenteneinsatz von dem Schaft gelöst wird; ferner kann der Handgriff vom Schaft getrennt werden.

Für die lösbare Verbindung des Instrumenteneinsatzes mit einem distalen Endabschnitt des Schafts ist insbesondere eine Verbindung nach Art einer Bajonettverbindung bekannt. Endoskopische Instrumente, bei denen ein Werkzeug bzw. ein Instrumenteneinsatz nach Art einer Bajonettverbindung mit dem distalen Ende eines Schafts verbindbar ist, sind beispielsweise in EP 0 688 187 B1 und DE 197 22 062 A1 offenbart. Dabei stellt eine Zugstange, über die das Werkzeug betätigt werden kann, zugleich eine Verdrehsicherung dar, um den Bajonetteingriff aufrecht zu erhalten. Hierfür ist die Zugstange im proximalen Bereich abgeflacht, wobei zwei Klemmstücke in die Abflachung eingreifen, so dass ein Verdrehen der Zugstange relativ zu einem Rohrschaft des Instruments verhindert wird. US 2012/259325 A1 offenbart ein endoskopisches Instrument gemäß der Präambel. US 2021/275203 A1, US 2017/360462 A1 und US 2002/111534 A1 offenbaren weitere ähnliche endoskopische Instrumente.

Gattungsgemäße medizinische endoskopische Instrumente werden von KARL STORZ SE & Co. KG beispielsweise unter den Bezeichnungen "ClickLine^{®}" sowie "RoBi^{®}" vertrieben.

Es ist Aufgabe der vorliegenden Erfindung, ein endoskopisches Instrument anzugeben, insbesondere ein endoskopisches medizinisches Instrument, wobei ein Instrumenteneinsatz durch einen Verbindungsmechanismus lösbar mit einem distalen Endabschnitt eines Schafts des Instruments verbunden ist, wobei insbesondere eine Verbindung ohne Verdrehsicherung oder mit einer alternativen Verdrehsicherung besteht.

Diese Aufgabe wird durch ein endoskopisches Instrument gemäß Anspruch 1 gelöst. Vorteilhafte Weiterbildungen der Erfindung ergeben sich aus den Unteransprüchen.

Ein erfindungsgemäßes endoskopisches Instrument ist insbesondere ein medizinisches endoskopisches Instrument, beispielsweise ein chirurgisches endoskopisches Instrument zur Durchführung von minimal-invasiven chirurgischen Eingriffen. Das endoskopische Instrument umfasst einen lang erstreckten Schaft, der insbesondere zur Einführung in einen körperinneren Hohlraum eines menschlichen oder tierischen Körpers ausgebildet ist. Der Schaft ist vorzugsweise starr oder im Wesentlichen starr ausgebildet und weist ein langerstrecktes Schaftrohr auf, das einen Außenschaft des Instruments darstellen kann; andererseits kann der Schaft aber auch flexibel ausgebildet sein. Weiter umfasst das endoskopische Instrument einen Instrumenteneinsatz, der mit einem distalen Endabschnitt des Schafts lösbar verbunden ist, insbesondere am distalen Ende des Schafts lösbar gehalten ist.

Der Instrumenteneinsatz umfasst ein Werkzeug, das insbesondere zur Ausführung endoskopischer Manipulationen in dem körperinneren Hohlraum ausgebildet ist, sowie eine vorzugsweise schaftförmige Basis, die auch als Gabel bezeichnet wird. Das Werkzeug kann zwei miteinander zusammenwirkende Werkzeugelemente aufweisen, von denen mindestens eines relativ zur Basis beweglich ist. Das Werkzeug kann beispielsweise eine chirurgische Schere oder Greifzange sein, die ein oder zwei relativ zueinander schwenkbare, als Scheren- bzw. Maulteile ausgebildete Werkzeugelemente aufweist, wovon mindestens eines gelenkig an der Basis gelagert ist. Das Werkzeug kann über ein langerstrecktes Übertragungselement betätigbar sein, das innerhalb des Schafts bis zu dessen proximalem Ende verläuft und welches beispielsweise als Zugstange ausgebildet sein kann, die gelenkig mit dem mindestens einen beweglichen Werkzeugelement verbunden ist. Das Übertragungselement kann ein Teil des Instrumenteneinsatzes sein.

Erfindungsgemäß ist der distale Endabschnitt des Schafts des endoskopischen Instruments als Hülse ausgebildet, und ein proximaler Endbereich der Basis des Instrumenteneinsatzes ist als Kupplungsschaft ausgebildet, wobei der Kupplungsschaft zum lösbaren Verbinden des Instrumenteneinsatzes mit dem Schaft lösbar in der Hülse gehalten ist. Alternativ erfindungsgemäß kann der proximale Endbereich der Basis des Instrumenteneinsatzes als Hülse und der distale Endabschnitt des Schafts als Kupplungsschaft ausgebildet sein, der zum lösbaren Verbinden des Instrumenteneinsatzes mit dem Schaft lösbar in der Hülse gehalten ist.

Die Hülse, die auch als Kupplungshülse bezeichnet werden kann, und der Kupplungsschaft, der auch als Kupplungszapfen bezeichnet werden kann, bilden somit eine Kupplung zum lösbaren Verbinden des Instrumenteneinsatzes mit dem Schaft und sind dementsprechend zum Zusammenwirken miteinander ausgestaltet.

Wenn der Instrumenteneinsatz mittels der durch die Hülse und den Kupplungsschaft gebildeten Kupplung mit dem Schaft verbunden ist, so sind die Basis und das Werkzeug vorzugsweise in einer distalseitigen Verlängerung einer Längsachse des Schafts angeordnet. Insbesondere kann die Basis eine Längsachse aufweisen, die dann, wenn der Instrumenteneinsatz mit dem Schaft verbunden ist, mit der Längsachse des Schafts zumindest näherungsweise fluchtet. Der Kupplungsschaft ist vorzugsweise als Hohlschaft bzw. Hohlrohr ausgebildet, durch welches das Übertragungselement hindurch verlaufen kann. In dem Fall, dass der distale Endabschnitt des Schafts als Hülse ausgebildet ist, kann die Hülse eine distale Verlängerung oder einen distalen Endabschnitt eines Schaftrohrs darstellen, wobei das Übertragungselement im Inneren des Schaftrohrs geführt ist.

Weiter erfindungsgemäß weist die Hülse und/oder der Kupplungsschaft zumindest abschnittsweise ein Querschnittsprofil auf, das zum Lösen des Kupplungsschafts aus der Hülse reversibel veränderbar ist. Die Hülse und/oder der Kupplungsschaft ist erfindungsgemäß somit, auf die Längsachse des Schafts bzw. der Basis bezogen, zumindest in einem Abschnitt mit einem veränderbaren Querschnittsprofil ausgebildet, und die Hülse und der Kupplungsschaft sind derart ausgestaltet, dass durch Veränderung des Querschnittsprofils der Kupplungsschaft aus der Hülse lösbar ist. Die Veränderung des Querschnittsprofils kann dabei eine Änderung der Form des Querschnitts sein, also eine Verformung des Querschnittsprofils bzw. der Querschnittskontur, und/oder eine Änderung der Dimensionen des Querschnitts, also eine Änderung der Größe bzw. des Radius des Querschnittsprofils bzw. der Querschnittskontur. Insbesondere kann die Hülse und/oder der Kupplungsschaft reversibel verformbar sein und derart ausgestaltet sein, dass durch eine Querschnittsverformung der Kupplungsschaft aus der Hülse gelöst werden kann. Die Querschnittsverformung, durch die die Kupplung gelöst wird, kann insbesondere durch eine von außen einwirkende Kraft bewirkt werden. Die von außen einwirkende Kraft kann manuell von einem Benutzer ausübbar sein, etwa eine in radialer Richtung auf die Hülse ausgeübte Druckkraft. Dabei ist eine Steifigkeit der Hülse bzw. des Kupplungsschafts insbesondere derart gewählt, dass die Drucckraft durch den Benutzer mit den Fingern einer Hand bequem ausgeübt werden kann, jedoch durch eine Berührung mit körperinneren Strukturen, wie etwa einer Wand des körperinneren Hohlraums, in der Regel nicht aufgebracht werden kann.

Der Schaft kann mit einer Umhüllung versehen sein, durch die beispielsweise eine Schutzfunktion oder eine elektrische Isolierung bereitgestellt wird. In dem Fall, dass der Schaft ein starres Schaftrohr umfasst, besteht das Schaftrohr vorzugsweise aus einem metallischen Material, etwa aus Edelstahl, und die Umhüllung aus einem Kunststoffmaterial, welches etwa in Form eines Schrumpfschlauchs auf der Außenseite des Schaftrohrs aufliegen kann. Das endoskopische Instrument kann beispielsweise ein elektrochirurgisches Instrument sein.

Das endoskopische Instrument kann ferner einen Handgriff umfassen mit einem beweglichen Teil, der mit einem proximalen Ende des Übertragungselements verbindbar sein kann, so dass das Werkzeug durch Betätigung des beweglichen Teils mittels einer Bewegung des Übertragungselements relativ zum Schaft betätigbar ist. Vorzugsweise ist der Handgriff mit einem proximalen Ende des Schafts lösbar verbindbar.

Dadurch, dass die Hülse und/oder der Kupplungsschaft durch reversible Änderung des Querschnittsprofils voneinander lösbar sind, wird es auf einfache Weise ermöglicht, die Kupplung zu lösen und somit den Instrumenteneinsatz vom Schaft zu lösen. Insbesondere kann die Querschnittsänderung eine Querschnittsverformung sein, die durch eine manuelle Krafteinwirkung von einem Benutzer bewirkt werden kann, wodurch ein besonders einfaches Trennen des Instrumenteneinsatzes vom Schaft ermöglicht werden kann. Zudem kann dadurch, dass zum Lösen der Kupplung eine Veränderung des Querschnittsprofils erforderlich ist, ein sicheres Halten des Instrumenteneinsatzes am Schaft ohne eine zusätzliche Verdrehsicherung ermöglicht werden. Somit kann ein Lösen des Instrumenteneinsatzes vom Schaft auf einfache Weise ermöglicht und zugleich ein unbeabsichtigtes Lösen sicher verhindert werden. Dadurch, dass das endoskopische Instrument zerlegbar ist und der Instrumenteneinsatz vom Schaft lösbar ist, können die Reinigung und Sterilisation des Instruments sowie ein Wechsel des Instrumenteneinsatzes erleichtert werden.

Im Folgenden wird die Erfindung insbesondere anhand von Ausführungsformen erläutert, bei denen der distale Endabschnitt des Schafts eine Hülse umfasst bzw. als solche ausgebildet ist, und wobei der Instrumenteneinsatz einen in die Hülse einführbaren Kupplungsschaft umfasst. Es versteht sich jedoch, dass jeweils auch, in entsprechend umgekehrter Anordnung, der Kupplungsschaft dem Schaft des endoskopischen Instruments und die Hülse dem Instrumenteneinsatz zugeordnet sein kann.

Das erfindungsgemäße endoskopische Instrument kann derart ausgebildet sein, dass zum Schließen der Kupplung ebenfalls eine reversible Veränderung des Querschnittsprofils erforderlich ist. Insbesondere kann der Kupplungsschaft zum Verbinden des Instrumenteneinsatzes mit dem Schaft in die Hülse einführbar sein, wobei zum Schließen der Kupplung eine Veränderung des Querschnittsprofils, etwa eine Querschnittsverformung oder Größenänderung des Querschnitts, erfolgen kann, die umgekehrt zu der zum Lösen der Kupplung erforderlichen Änderung sein kann. Hierdurch kann auf einfache Weise eine feste und sichere Verbindung des Instrumenteneinsatzes mit dem Schaft erreichbar sein.

Erfindungsgemäß ist die reversible Veränderung des Querschnittsprofils zum Lösen des Kupplungsschafts aus der Hülse eine reversible Verformung des Querschnittsprofils der Hülse und/oder des Kupplungsschafts, insbesondere eine elastische Verformung. Die Hülse bzw. der Kupplungsschaft ist somit zum Lösen der Verbindung elastisch verformbar ausgebildet. Zum Lösen der Verbindung des Instrumenteneinsatzes mit dem Schaft ist daher eine Querschnittsverformung der Hülse bzw. des Kupplungsschafts gegen eine elastische Rückstellkraft erforderlich. Insbesondere kann die Hülse bzw. der Kupplungsschaft zum Halten des Kupplungsschafts in der Hülse vorgespannt sein. Dies hat den Vorteil, dass ohne Einwirkung einer äußeren Kraft die Kupplung zwangsgeschlossen ist und ferner der Kupplungsschaft besonders sicher in der Hülse gehalten werden kann; weiterhin kann hierdurch das Schließen der Kupplung erleichtert werden.

Insbesondere kann das Querschnittsprofil ein im Wesentlichen geschlossenes Querschnittsprofil sein, d.h. das reversibel veränderbare Querschnittsprofil ist in Umfangsrichtung im Wesentlichen geschlossen ausgebildet, beispielsweise ringförmig geschlossen. Die Hülse und/oder der Kupplungsschaft weist somit gemäß dieser Ausführungsform zumindest abschnittsweise ein im Wesentlichen geschlossenes Querschnittsprofil auf, das zum Lösen des Kupplungsschafts aus der Hülse reversibel veränderbar ist, insbesondere durch elastische Verformung reversibel veränderbar ist. "Im Wesentlichen geschlossen" bedeutet hier und im Folgenden insbesondere, dass der Abschnitt der Hülse bzw. des Kupplungsschafts, in dem das Querschnittsprofil reversibel verformbar ist, zumindest einen Teilabschnitt umfasst, in dem das Querschnittsprofil umlaufend geschlossen ist, beispielsweise ringförmig geschlossen. Hierdurch können eine besonders feste Verbindung und eine hohe Biegebelastbarkeit der Verbindung des Instrumenteneinsatzes mit dem distalen Endbereich des Schafts erreichbar sein.

Gemäß der Erfindung weist die Hülse eine erste Haltestruktur und der Kupplungsschaft eine zweite Haltestruktur auf, die mit der ersten Haltestruktur zum Halten des Kupplungsschafts in der Hülse zusammenwirkt. Die erste und die zweite Haltestruktur sind somit derart zum Zusammenwirken ausgestaltet, dass der Kupplungsschaft in der Hülse gehalten ist und insbesondere gegen Lösen in axialer Richtung gesichert ist, so dass der Instrumenteneinsatz mit dem Schaft verbunden ist. Weiterhin ist gemäß dieser Ausführungsform die Hülse durch reversible, insbesondere elastische Querschnittsverformung aus einer Arbeitsform, in der die erste Haltestruktur und die zweite Haltestruktur zum Halten des Kupplungsschafts in der Hülse zusammenwirken, in eine Montageform, in der die erste Haltestruktur und die zweite Haltestruktur voneinander gelöst sind, überführbar. Alternativ oder zusätzlich kann der Kupplungsschaft durch reversible, insbesondere elastische Querschnittsverformung aus einer Arbeitsform, in der die erste und die zweite Haltestruktur zum Halten des Kupplungsschafts in der Hülse zusammenwirken, in eine Montageform, in der die erste und die zweite Haltestruktur voneinander gelöst sind, überführbar sein. Zum Halten der Verbindung weisen die Hülse bzw. der Kupplungsschaft somit eine jeweilige Arbeitsform auf, in der miteinander korrespondierende Haltestrukturen zum Halten zusammenwirken, wobei zum Lösen der Verbindung die Hülse und/oder der Kupplungsschaft durch reversible Verformung in eine Montageform gebracht werden können. Insbesondere ist die erste und/oder die zweite Haltestruktur in der jeweiligen Arbeitsform zum Zusammenwirken mit der jeweils korrespondierenden Haltestruktur angeordnet und in der Montageform von dieser getrennt angeordnet. Wenn die Hülse bzw. der Kupplungsschaft durch reversible Verformung in die Montageform überführt worden ist, ist der Kupplungsschaft somit aus der Hülse entnehmbar. Zum Einführen des Kupplungsschafts in die Hülse, d.h. zum Verbinden des Instrumenteneinsatzes mit dem Schaft bzw. zum Schließen der Kupplung kann die Hülse und/oder der Kupplungsschaft aus der Montageform in die Arbeitsform bringbar sein. Die Hülse und/oder der Kupplungsschaft kann insbesondere im Bereich der Haltestruktur einen geschlossenen Querschnitt haben.

Dadurch, dass die Hülse eine erste Haltestruktur und der Kupplungsschaft eine zweite Haltestruktur aufweisen, wobei die zweite mit der ersten Haltestruktur zum Halten des Kupplungsschafts in der Hülse zusammenwirkt, kann, wenn die Hülse bzw. der Kupplungsschaft die Arbeitsform hat, ein sicheres Halten des Instrumenteneinsatzes am Schaft und damit ein sicheres Arbeiten mit dem endoskopischen Instrument ermöglicht werden. Ferner kann, wenn die Hülse bzw. der Kupplungsschaft die Montageform hat, ein einfaches Trennen des Instrumenteneinsatzes vom Schaft des Instruments ermöglicht werden, beispielsweise zur Reinigung oder für einen Wechsel des Instrumenteneinsatzes. In dem Fall, dass die Hülse bzw. der Kupplungsschaft durch elastische Querschnittsverformung aus der Arbeitsform in die Montageform überführbar ist, kann erreicht werden, dass ohne Einwirkung von außen die Arbeitsform erhalten und somit die Kupplung sicher geschlossen bleibt, dass durch eine temporäre Querschnittsverformung durch äußere Krafteinwirkung gegen eine elastische Rückstellkraft die Kupplung geöffnet werden kann, und dass, wenn keine äußere Kraft mehr einwirkt, durch elastische Rückverformung das Querschnittsprofil wieder die Arbeitsform einnimmt.

Der Schaft kann in vorteilhafter Weise einen Querschnitt mit einer Außenkontur haben, die der Querschnittskontur der Hülse in der Arbeitsform entspricht, oder beispielsweise einen kreisförmigen Querschnitt, der in einem Übergangsabschnitt der Hülse und/oder des Schafts in den Querschnitt der Hülse in der Arbeitsform übergeht. Sofern der Schaft starr ausgebildet ist, kann insbesondere ein starres Schaftrohr, das einen Außenschaft bildet, einen Querschnitt mit einer solchen Kontur haben, die der Querschnittskontur der Hülse in der Arbeitsform entspricht oder in diese übergeht. Insbesondere kann der Schaftrohr eine größere Wandstärke als die Hülse haben, jedoch eine Querschnittskontur, die der Querschnittskontur der Hülse in der Arbeitsform entspricht oder im Übergangsabschnitt in diese übergeht; das Schaftrohr kann beispielsweise ein Profilrohr mit einem entsprechenden Querschnittsprofil sein. Hierdurch wird es ermöglicht, dass der Schaft des endoskopischen Instruments eine weitgehend glatte Außenkontur hat.

Gemäß der Erfindung erstreckt sich die erste Haltestruktur in Umfangsrichtung über einen oder mehrere Teil-Winkelbereiche der Hülse .

Die erste Haltestruktur nimmt somit, von der Längsachse der Hülse bzw. des Kupplungsschafts aus gesehen, einen oder mehrere Teil-Winkelbereiche ein, wobei jedoch mindestens ein freier Winkelbereich nicht von der ersten Haltestruktur eingenommen wird. Alternativ oder zusätzlich kann es vorgesehen sein, dass sich die zweite Haltestruktur, auf die Längsachse bezogen, über einen oder mehrere Teil-Winkelbereiche des Kupplungsschafts erstreckt, wobei mindestens ein freier Winkelbereich nicht von der zweiten Haltestruktur eingenommen wird. Weiter weist die Hülse mindestens einen Druckpunkt zum Verformen des Querschnittsprofils der Hülse aus der Arbeitsform in die Montageform auf, wobei der mindestens eine Druckpunkt außerhalb der Teil-Winkelbereiche der ersten bzw. der zweiten Haltestruktur gelegen ist, d.h. in dem mindestens einen freien Winkelbereich. Der mindestens eine Druckpunkt ist somit gegenüber der ersten bzw. der zweiten Haltestruktur versetzt und kann beispielsweise etwa in der Mitte eines freien Winkelbereichs angeordnet sein. Vorzugsweise ist der mindestens eine Druckpunkt in einem Winkelbereich gelegen, in dem das Querschnittsprofil der Hülse eine konvexe Form hat. In besonders vorteilhafter Weise kann das Querschnittsprofil der Hülse in mindestens einem Abschnitt im Bereich der ersten Haltestruktur ringförmig geschlossen und umlaufend konvex ausgebildet sein. Die Hülse und der Kupplungsschaft sind insbesondere derart ausgebildet, dass durch Einwirkung einer Druckkraft von außen in radialer Richtung das Querschnittsprofil der Hülse in einer solchen Weise verformt wird, dass die erste Haltestruktur in radialer Richtung angehoben und dadurch von der zweiten Haltestruktur getrennt wird und nicht mehr mit der zweiten Haltestruktur zum Halten des Kupplungsschafts in der Hülse zusammenwirkt. Hierfür kann zwischen einer äußeren Oberfläche des Kupplungsschafts und einer inneren Oberfläche der Hülse im Bereich des Druckpunkts ein Hohlraum vorgesehen sein, um die Hülse im Bereich des Druckpunkts ausreichend zusammendrücken zu können.

Gemäß einer Ausführungsform der Erfindung ist die Arbeitsform der Hülse eine abgeflachte oder abgeplattete Form, etwa eine elliptische oder ovale Form, wobei in den Scheitelpunkten der abgeflachten Querschnittsform jeweils ein Druckpunkt angeordnet ist und somit zwei, bezüglich der Längsachse zumindest näherungsweise einander gegenüberliegende Druckpunkte vorgesehen sind. Der Querschnitt der Hülse hat somit zumindest im Bereich der ersten Haltestruktur eine umlaufend konvexe Außenkontur, wobei im Bereich der einander gegenüberliegenden Scheitelpunkte die Außenkontur jeweils eine größere Krümmung aufweist als in den dazwischenliegenden Bereichen, und wobei die Druckpunkte näherungsweise in den Bereichen der größeren Krümmung angeordnet sind. Durch Ausüben einer Druckkraft auf die Druckpunkte kann die abgeflachte Form von den Scheitelpunkten her zusammengedrückt werden, so dass die zwischen den Scheitelpunkten liegenden flacheren Bereiche, in denen die erste oder die zweite Haltestruktur angeordnet ist, zumindest teilweise nach außen verlagert werden und dadurch das Querschnittsprofil die Montageform einnimmt, in der die erste von der zweiten Haltestruktur abgehoben ist. Die Montageform kann beispielsweise ebenfalls eine abgeflachte Form sein, wobei die Hauptachse, die die Scheitelpunkte verbindet, gegenüber der Arbeitsform um etwa 90° gedreht ist, kann aber auch eine Kreisform oder eine abgeflachte Form mit derselben Hauptachse wie die Arbeitsform, jedoch mit geringerer Abplattung bzw. Elliptizität sein. Hierdurch kann ein unbeabsichtigtes Lösen sicher verhindert werden und dennoch die Kupplung bequem lösbar sein.

Alternativ kann die Arbeitsform eine Kreisform sein, wobei die beiden Druckpunkte einander gegenüberliegend angeordnet sind, und die Montageform ist eine abgeflachte, etwa elliptische oder ovale Form, bei der die Druckpunkte in Umfangsrichtung zwischen den Scheitelpunkten, insbesondere etwa mittig zwischen den Scheitelpunkten, liegen. Weiter alternativ kann die Arbeitsform eine abgeflachte Form sein, wobei die Druckpunkte zwischen den Scheitelpunkten angeordnet sind, wobei die Montageform eine abgeflachte Form mit gleicher Hauptachse, jedoch größerer Abplattung bzw. Elliptizität, ist. Auch hierdurch kann ein einfaches Lösen der Kupplung durch manuelle Ausübung einer Druckkraft auf die einander gegenüber liegenden Druckpunkte ermöglicht werden.

Gemäß einer weiteren Ausführungsform der Erfindung weist die Hülse drei Druckpunkte auf, die bezüglich der Längsachse der Hülse jeweils um etwa 120° gegeneinander versetzt sind, wobei das Querschnittsprofil bzw. die Querschnittskontur ebenfalls vorzugsweise umlaufend konvex ist. Durch Ausübung einer Druckkraft auf die drei Druckpunkte können die jeweils dazwischen liegenden Bereiche der Querschnittsform in radialer Richtung angehoben werden, so dass die erste und die zweite Haltestruktur dort voneinander getrennt werden. Auch hierdurch kann auf einfache Weise ein manuelles Lösen der Verbindung ermöglicht werden, wobei eine besonders hohe Sicherheit gegen ein unbeabsichtigtes Lösen erreichbar ist.

Vorzugsweise sind die Hülse und/oder der Kupplungsschaft derart ausgebildet, dass diese durch Ausüben einer Druckkraft auf den mindestens einen Druckpunkt mit einem oder mehreren Fingern einer Hand durch einen Benutzer voneinander lösbar sind, wobei die Drucckraft bequem mit den Fingern, jedoch in der Regel nicht durch Berührung mit körperinneren Strukturen aufgebracht werden kann. Insbesondere kann die Druckkraft mit zwei Fingern einer Hand auf zwei gegenüber liegende Druckpunkte oder mit drei Fingern auf drei um 120° versetzte Druckpunkte ausübbar sein. Dabei kann eine Druckkraft beispielsweise im Bereich von etwa 1 bis 30 N, vorzugsweise im Bereich von ca. 3 bis 10 N, insbesondere etwa 5 N, zum Lösen des Kupplungsschafts aus der Hülse geeignet sein.

Die Hülse bzw. der Kupplungsschaft kann insgesamt oder lediglich in einem oder mehreren Abschnitten das reversibel verformbare Querschnittsprofil aufweisen. Insbesondere kann die Hülse in einem Abschnitt, der bis an ein distales Ende der Hülse reicht, bzw. der Kupplungsschaft in einem Abschnitt, der bis an ein proximales Ende des Kupplungsschafts reicht, zum Lösen des Kupplungsschafts aus der Hülse einen reversibel verformbaren Querschnitt haben. Andererseits kann es beispielsweise auch vorgesehen sein, dass die Hülse bzw. der Kupplungsschaft nur in einem mittleren Abschnitt zum Lösen des Kupplungsschafts von der Hülse verformt wird, während ein proximales und ein distales Ende nicht verformt werden; in diesem Fall sind der bzw. die Druckpunkte im mittleren Abschnitt angeordnet.

Gemäß einer vorteilhaften Ausführungsform weist der Kupplungsschaft einen Stützabschnitt zur Abstützung eines Endabschnitts der Hülse in der Arbeitsform auf. Der Endabschnitt der Hülse ist in dem Fall, dass die Hülse dem Schaft zugeordnet ist, ein distaler Endabschnitt und in dem Fall, dass die Hülse dem Instrumenteneinsatz zugeordnet ist, ein proximaler Endabschnitt. Der Stützabschnitt kann beispielsweise im ersten Fall im distalen und im zweiten Fall im proximalen Endbereich des Kupplungsschafts angeordnet sein. Der Stützabschnitt weist insbesondere eine Außenkontur auf, die zumindest näherungsweise der Innenkontur der Hülse im entsprechenden Endabschnitt in der Arbeitsform entspricht. Dadurch wird die Hülse in der Arbeitsform in ihrem entsprechenden Endabschnitt abgestützt, so dass die Hülse nur in einem mittleren Abschnitt zum Lösen des Kupplungsschafts von der Hülse verformt werden kann. Hierdurch kann erreicht werden, dass trotz des zur Verformung notwendigen Hohlraums innerhalb der Hülse die Kupplung eine hohe Steifigkeit gegen seitliche Belastung auch in der entsprechenden Richtung aufweist.

In vorteilhafter Weise kann die erste Haltestruktur als Eingriffsstruktur und die zweite Haltestruktur als zum Eingreifen in die erste Haltestruktur ausgebildetes Eingriffselement ausgebildet sein, oder umgekehrt kann die zweite Haltestruktur als Eingriffsstruktur und die erste Haltestruktur als zum Eingreifen in die zweite Haltestruktur ausgebildetes Eingriffselement ausgebildet sein. Die Eingriffsstruktur weist insbesondere, aus distaler axialer Richtung gesehen, einen Hinterschnitt auf und kann beispielsweise durch einen in Umfangsrichtung verlaufenden Schlitz oder Einstich bzw. durch eine in Umfangsrichtung verlaufende Nut gebildet werden. Das Eingriffselement ist insbesondere in radialer Richtung von der Hülse nach innen bzw. von dem Kupplungsschaft nach außen vorstehend angeordnet und kann insbesondere als Ringbund oder Nocken, beispielsweise als Bajonettelement, ausgebildet sein; das Eingriffselement kann somit, aus proximaler axialer Richtung gesehen, einen Hinterschnitt aufweisen. In der Arbeitsform greift somit das Eingriffselement in die Eingriffsstruktur ein, so dass der Kupplungsschaft in der Hülse gehalten ist. In der Montageform ist das Eingriffselement aus der Eingriffsstruktur gelöst, so dass der Kupplungsschaft aus der Hülse entnehmbar ist. In besonders vorteilhafter Weise kann die Hülse und/oder der Kupplungsschaft zumindest in einem Abschnitt, in dem die Eingriffsstruktur bzw. das Eingriffselement angeordnet ist, ein ggf. bis auf die Eingriffsstruktur selbst geschlossenes Querschnittsprofil haben. Hierdurch kann eine besonders feste, insbesondere eine formschlüssige Verbindung der Basis des Instrumenteneinsatzes mit dem Schaft erreicht werden.

Gemäß einer Ausführungsform der Erfindung weist die zweite Haltestruktur mindestens einen radial über eine Oberfläche des Kupplungsschafts vorstehenden Nocken auf, und die erste Haltestruktur weist mindestens einen hiermit korrespondierenden Durchbruch oder einen in Umfangsrichtung verlaufenden Schlitz oder auch eine in Umfangsrichtung verlaufende Nut auf, wobei der mindestens eine Nocken zum Eingreifen in den korrespondierenden Durchbruch bzw. Schlitz ausgebildet und angeordnet ist. Der Nocken stellt somit ein Eingriffselement und der Durchbruch bzw. der Schlitz oder die Nut eine entsprechende Eingriffsstruktur dar. Insbesondere kann die Arbeitsform der Hülse und/oder des Kupplungsschafts derart ausgestaltet sein, dass der mindestens eine Nocken in den Durchbruch bzw. Schlitz eingreift und dadurch der Kupplungsschaft in der Hülse gehalten wird, und die Montageform der Hülse und/oder des Kupplungsschafts derart, dass der mindestens eine Nocken nicht in den Durchbruch bzw. Schlitz eingreift, so dass der Kupplungsschaft aus der Hülse lösbar ist. Gemäß einer Variante dieser Ausführungsform greift der mindestens eine Nocken derart in die zweite Haltestruktur ein, dass der Nocken von einer Außenseite der Hülse aus manuell eindrückbar ist und hierdurch der Kupplungsschaft in die Montageform überführt werden kann. Hierdurch kann eine besonders hohe Sicherheit gegen ein unbeabsichtigtes Lösen der Kupplung erreichbar sein.

Gemäß einer vorteilhaften Ausführungsform der Erfindung wirken die erste und die zweite Haltestruktur zum drehfesten Halten des Kupplungsschafts in der Hülse zusammen. Die erste und die zweite Haltestruktur sind insbesondere derart ausgebildet, dass dann, wenn das Eingriffselement in die Eingriffsstruktur eingreift, der Kupplungsschaft nicht nur gegen ein Entnehmen aus der Hülse in axialer Richtung, sondern auch gegen eine Rotation um die Längsachse gehalten ist. Hierfür können beispielsweise ein als radial vorstehender Nocken ausgebildetes Eingriffselement und eine als in Umfangsrichtung verlaufende Nut ausgebildete Eingriffsstruktur einander entsprechende Erstreckungen in Umfangsrichtung haben. Diese können beispielsweise auch derart aufeinander abgestimmt sein, dass eine Rotation um einen begrenzten Drehwinkel zugelassen ist und beiderseitig entsprechende Anschläge in Umfangsrichtung vorgesehen sind. Hierdurch kann es erreicht werden, dass das Werkzeug besonders fest mit dem Schaft verbunden ist und eine durch Rotation des Schafts um seine Längsachse kontrollierbare Ausrichtung aufweist. Alternativ kann aber auch durch umlaufende Gestaltung der Eingriffsstruktur und/oder des Eingriffselements eine freie Drehbarkeit des Kupplungsschafts um die Längsachse erreichbar sein.

Gemäß einer weiteren Ausführungsform der Erfindung weisen die erste und/oder die zweite Haltestruktur in Umfangsrichtung ein- oder beidseitig eine Anlaufschräge auf. Durch Rotation der Hülse relativ zum Kupplungsschaft kann somit über die entsprechende Anlaufschräge die erste Haltestruktur in radialer Richtung angehoben und/oder die zweite Haltestruktur in radialer Richtung eingedrückt werden. Auf diese Weise kann durch Rotation der Hülse relativ zum Kupplungsschaft die Hülse und/oder der Kupplungsschaft aus der Arbeitsform in die Montageform überführt werden. Dies hat den weiteren Vorteil, dass durch eine Rotation und eine nachfolgende axiale Bewegung der Basis des Instrumenteneinsatzes relativ zum Schaft der Instrumenteneinsatz vom Schaft gelöst werden kann, was etwa der Bewegung beim Lösen einer Bajonettverbindung entspricht. Insbesondere können die Hülse und der Kupplungsschaft derart ausgestaltet sein, dass beim Verbinden der Kupplung durch die Rotationsbewegung eine Verformung des Querschnittsprofils des Kupplungsschafts bzw. der Hülse erzwungen wird und nach Erreichen einer entsprechenden Winkelstellung der Kupplungsschaft und die Hülse miteinander verrasten. Hierdurch kann daher eine Verbindung geschaffen werden, die besonders einfach zu lösen ist und zugleich weitestgehend gegen unbeabsichtigtes Lösen gesichert ist.

Vorzugsweise weist der Kupplungsschaft eine federnde Lasche auf und die Hülse einen Durchbruch, in den eine Rastnase der Lasche zum drehfesten Halten des Kupplungsschafts in der Hülse eingreifen kann. Insbesondere kann die Rastnase derart in den Durchbruch eingreifen, dass diese von außen manuell eingedrückt werden kann, so dass die Hülse relativ zum Kupplungsschaft nach Eindrücken der Rastnase verdrehbar ist. Insbesondere bei der vorgenannten Ausführungsform, bei der die Kupplung durch eine Rotation der Hülse relativ zur Basis des Instrumenteneinsatzes gelöst werden kann, kann hierdurch eine weiter erhöhte Sicherheit gegen unbeabsichtigtes Lösen erreicht werden.

In vorteilhafter Weise kann es vorgesehen sein, dass die erste und/oder die zweite Haltestruktur in axialer Richtung in einer Einführrichtung eine Anlaufschräge aufweist. Eine derartige Einlaufgeometrie kann beispielsweise durch eine in derjenigen axialen Richtung, in der zum Schließen der Kupplung der Kupplungsschaft in die Hülse eingeführt wird, ansteigende Rampe oder entsprechende Flügel gebildet werden. Über die Anlaufschräge kann beim Einführen des Kupplungsschafts das Querschnittsprofil des Kupplungsschafts und/oder der Hülse beispielsweise derart verformt werden, insbesondere gegen eine elastische Rückstellkraft, dass die Kupplung ohne Ausübung einer äußeren Kraft auf die Hülse geschlossen werden kann und lediglich zum Lösen der Kupplung eine äußere Krafteinwirkung notwendig ist. Insbesondere können die Hülse und der Kupplungsschaft derart ausgestaltet sein, dass beim Verbinden der Kupplung durch die axiale Bewegung eine Verformung des Querschnittsprofils des Kupplungsschafts bzw. der Hülse erzwungen wird und nach Erreichen einer entsprechenden axialen Position der Kupplungsschaft und die Hülse miteinander verrasten. Hierdurch kann die Montage des endoskopischen Instruments weiter erleichtert werden.

Gemäß einer weiteren Ausführungsform der Erfindung können die erste und die zweite Haltestruktur miteinander zusammenwirkende Reibflächen umfassen und/oder für eine Mikroverzahnung ausgebildet sein. Durch die Reibflächen bzw. durch die Mikroverzahnung kann es erreicht werden, dass der Kupplungsschaft kraftschlüssig in der Hülse gehalten wird. Hierdurch kann ebenfalls der Instrumenteneinsatz einfach und sicher am Schaft gehalten und von diesem lösbar sein. Eine derartige Verbindung mittels Reibung bzw. Mikroverzahnung kann insbesondere alternativ oder zusätzlich zu der zuvor beschriebenen Verbindung mittels der Eingriffselemente und Eingriffsstrukturen vorgesehen sein.

Vorzugsweise besteht die Hülse und/oder der Kupplungsschaft zumindest teilweise aus metallischem Glas. Insbesondere kann der reversibel verformbare Abschnitt der Hülse bzw. des Kupplungsschafts aus metallischem Glas gefertigt sein. Durch die Verwendung von metallischem Glas können eine hohe elastische Verformbarkeit und zugleich eine hohe Dauerhaltbarkeit erreichbar sein. Alternativ kann die Hülse und/oder der Kupplungsschaft beispielsweise zumindest zum Teil aus Federstahl bestehen.

Gemäß einer weiteren Ausführungsform der Erfindung ist das Querschnittsprofil zumindest der Hülse zum Lösen des Kupplungsschafts aus der Hülse durch Temperatureinwirkung veränderbar. Gemäß dieser Ausführungsform kann die Verbindung zwischen der Basis des Instrumenteneinsatzes und dem Schaft beispielsweise nach Art eines Schrumpfspannfutters ausgestaltet sein. Insbesondere kann es vorgesehen sein, dass zumindest in dem Abschnitt, in dem das Querschnittsprofil reversibel veränderbar ist, die Hülse und der Kupplungsschaft jeweils näherungsweise zylindrisch ausgebildet sind, wobei der Außenradius des Kupplungsschafts und der Innenradius der Hülse derart gewählt sind, dass der Kupplungsschaft fest in der Hülse gehalten ist, wenn sowohl der Kupplungsschaft als auch die Hülse eine Arbeitstemperatur im Bereich zwischen etwa 30°C und 40°C haben, und dass der Kupplungsschaft aus der Hülse durch Erwärmung der Hülse auf eine Montagetemperatur lösbar ist. Auch hierdurch kann eine sichere, lösbare Verbindung des Instrumenteneinsatzes mit dem Schaft erreichbar sein.

Eine erfindungsgemäße Kupplung zum lösbaren Verbinden eines Instrumenteneinsatzes mit dem Schaft eines endoskopischen Instruments umfasst zwei miteinander zusammenwirkende Kupplungsteile, nämlich eine Kupplungshülse, die hier kurz als Hülse bezeichnet wird, und einen Kupplungsschaft, die lösbar miteinander verbindbar sind. Die Hülse kann an einem distalen Endabschnitt des Schafts und der Kupplungsschaft an einem proximalen Endbereich einer Basis des Instrumenteneinsatzes oder der Kupplungsschaft an dem distalen Endabschnitt des Schafts und die Hülse an einem proximalen Endbereich der Basis anordenbar sein oder einen solchen darstellen. Die Hülse und/oder der Kupplungsschaft weist zumindest abschnittsweise ein vorzugsweise im Wesentlichen geschlossenes Querschnittsprofil auf, das zum Lösen des Kupplungsschafts aus der Hülse reversibel veränderbar, insbesondere durch elastische Verformung veränderbar ist. Beispielsweise kann die Hülse eine erste und der Kupplungsschaft eine zweite Haltestruktur aufweisen, wobei die Hülse und/oder der Kupplungsschaft durch reversible Verformung aus einer jeweiligen Arbeitsform, in der die erste Haltestruktur mit der zweiten Haltestruktur zum Halten des Kupplungsschafts in der Hülse angeordnet ist, in eine jeweilige Montageform, in der die erste Haltestruktur und die zweite Haltestruktur voneinander getrennt angeordnet sind, überführbar ist. Insbesondere können die Hülse und der Kupplungsschaft wie oben beschrieben ausgebildet sein. Hierdurch können eine sichere Verbindung der beiden Kupplungsteile, wobei keine zusätzliche Verdrehsicherung notwendig ist, und ein einfaches Lösen der Kupplungsteile voneinander ermöglicht werden.

Die vorliegende Offenbarung betrifft weiterhin einen Schaft für ein endoskopisches Instrument sowie ferner einen Instrumenteneinsatz für ein endoskopisches Instrument. Das endoskopische Instrument ist insbesondere ein medizinisches endoskopisches Instrument, beispielsweise ein chirurgisches endoskopisches Instrument.

Ein Schaft ist lang erstreckt und insbesondere zur Einführung in einen körperinneren Hohlraum eines menschlichen oder tierischen Körpers ausgestaltet. Der Schaft ist vorzugsweise starr oder im Wesentlichen starr und weist ein langerstrecktes Schaftrohr auf, kann aber auch flexibel ausgebildet sein. Der Schaft kann an seinem proximalen Ende mit einem Handgriff verbindbar sein oder einen solchen umfassen. Der distale Endabschnitt des Schafts des endoskopischen Instruments ist als Hülse oder als Kupplungsschaft ausgebildet, wobei die Hülse bzw. der Kupplungsschaft zumindest abschnittsweise ein vorzugsweise im Wesentlichen geschlossenes Querschnittsprofil aufweist, das reversibel verformbar ist, insbesondere durch elastische Verformung. Insbesondere kann die Hülse bzw. der Kupplungsschaft wie oben mit Bezug auf eine reversible Verformung des Querschnittsprofils beschrieben ausgebildet sein. Hierdurch kann auf einfache Weise erreicht werden, dass ein Instrumenteneinsatz eines endoskopischen Instruments, welches wie oben beschrieben ausgebildet sein kann, mit dem Schaft lösbar verbindbar ist.

Gemäß beispielhaften Ausgestaltungen des Schafts kann die Hülse bzw der Kupplungsschaft eine Haltestruktur aufweisen, wobei die Hülse bzw. der Kupplungsschaft durch reversible, insbesondere elastische Verformung aus einer Arbeitsform, in der die Haltestruktur zum Zusammenwirken mit einer korrespondierenden Haltestruktur eines Instrumenteneinsatzes des endoskopischen Instruments angeordnet ist, in eine Montageform, in der die Haltestruktur von der korrespondierenden Haltestruktur des Instrumenteneinsatzes getrennt angeordnet ist, überführbar ist. So kann etwa in dem Fall, dass der distale Endabschnitt des Schafts als Hülse ausgebildet ist, die Haltestruktur der Hülse in der Montageform in radialer Richtung mit größerem Abstand von einer Längsachse der Hülse angeordnet sein als in der Arbeitsform. Ferner kann in dem Fall, dass der distale Endabschnitt des Schafts als Kupplungsschaft ausgebildet ist, die Haltestruktur des Kupplungsschafts in der Montageform in radialer Richtung mit kleinerem Abstand von der Längsachse der Hülse angeordnet sein als in der Arbeitsform.

Ein Instrumenteneinsatz umfasst ein Werkzeug, das insbesondere zur Ausführung endoskopischer Manipulationen in einem körperinneren Hohlraum ausgebildet ist, sowie eine vorzugsweise schaftförmige Basis. Das Werkzeug umfasst vorzugsweise mindestens ein relativ zu der Basis bewegliches Werkzeugelement. Der Instrumenteneinsatz kann ein langerstrecktes Übertragungselement umfassen, mittels dessen das Werkzeug betätigbar ist. Ein proximaler Endbereich der Basis des Instrumenteneinsatzes ist als Hülse oder als Kupplungsschaft ausgebildet, wobei die Hülse bzw. der Kupplungsschaft zumindest abschnittsweise ein insbesondere im Wesentlichen geschlossenes Querschnittsprofil aufweist, das reversibel verformbar ist, insbesondere durch elastische Verformung. Insbesondere kann die Hülse bzw. der Kupplungsschaft wie oben mit Bezug auf eine reversible Verformung des Querschnittsprofils beschrieben ausgebildet sein. Hierdurch kann auf einfache Weise erreicht werden, dass der Instrumenteneinsatz mit einem Schaft eines endoskopischen Instruments, welcher wie oben beschrieben ausgebildet sein kann, lösbar verbindbar ist.

Gemäß beispielhaften Ausgestaltungen des Instrumenteneinsatzes kann die Hülse bzw. der Kupplungsschaft eine Haltestruktur aufweisen, wobei die Hülse bzw. der Kupplungsschaft durch reversible, insbesondere elastische Verformung aus einer Arbeitsform, in der die Haltestruktur zum Zusammenwirken mit einer korrespondierenden Haltestruktur eines Schafts des endoskopischen Instruments angeordnet ist, in eine Montageform, in der die Haltestruktur von der korrespondierenden Haltestruktur des Schafts getrennt angeordnet ist, überführbar ist. Beispielsweise kann in dem Fall, dass der proximale Endbereich der Basis als Hülse ausgebildet ist, die Haltestruktur der Hülse in der Montageform in radialer Richtung mit größerem Abstand von einer Längsachse der Hülse angeordnet sein als in der Arbeitsform. Ferner kann beispielsweise in dem Fall, dass der proximale Endbereich der Basis als Kupplungsschaft ausgebildet ist, die Haltestruktur des Kupplungsschafts in der Montageform in radialer Richtung mit kleinerem Abstand von der Längsachse der Hülse angeordnet sein als in der Arbeitsform.

Gemäß einem Verfahren zum Zerlegen eines endoskopischen Instruments, das wie oben beschrieben ausgebildet ist und bei dem die Hülse und/oder der Kupplungsschaft durch reversible Verformung aus einer jeweiligen Arbeitsform in eine jeweilige Montageform überführbar ist, wird durch äußere Krafteinwirkung der Querschnitt der Hülse und/oder des Kupplungsschafts aus der Arbeitsform in die Montageform überführt. Insbesondere kann durch Ausüben einer Druckkraft in radialer Richtung auf einen oder mehrere Druckpunkte der Querschnitt der Hülse derart verformt werden, dass die miteinander zum Halten des Kupplungsschafts in der Hülse zusammenwirkenden Haltestrukturen voneinander getrennt werden. Alternativ können beispielsweise durch Drehen der Hülse über in Umfangsrichtung gerichtete Anlaufschrägen die Hülse und/oder der Kupplungsschaft derart verformt werden, dass die korrespondierenden Haltestrukturen voneinander abgehoben werden. Der Kupplungsschaft wird sodann aus der Hülse in axialer Richtung entnommen und dadurch der Instrumenteneinsatz vom Schaft getrennt; ggf. wird dabei ein langerstrecktes Übertragungselement aus dem Schaft herausgezogen. Das Verfahren kann weitere Schritte umfassen, etwa das Abnehmen eines Handgriffs. Hierdurch kann das endoskopische Instrument nach einer Verwendung zerlegt werden, um es zu reinigen und zu sterilisieren, oder es kann ein weiterer Instrumenteneinsatz mit dem Schaft verbunden werden.

Zum Bereitstellen des endoskopischen Instruments vor einer Verwendung können ein Instrumenteneinsatz und ein Schaft des endoskopischen Instruments, die wie oben beschrieben ausgebildet sind, durch axiale Bewegung miteinander verbunden werden, wobei der Kupplungsschaft in die Hülse eingeführt wird und beispielsweise eine Haltestruktur des Instrumenteneinsatzes und eine Haltestruktur des Schafts miteinander in Eingriff gebracht werden; ggf. wird dabei zugleich ein langerstrecktes Übertragungselement des Instrumenteneinsatzes in den Schaft eingeführt. Für die Einführung des Kupplungsschafts in die Hülse kann eine Querschnittsverformung der Hülse und/oder des Kupplungsschafts durch manuelle Krafteinwirkung von außen erforderlich sein, so dass eine Montageform erreicht wird; in dem Fall, dass zumindest eine der Haltestrukturen in der Einführrichtung eine Anlaufschräge aufweist, kann hierfür die axiale Bewegung ausreichend sein, wobei eine Querschnittsverformung in die Montageform erzwungen wird. Nach Erreichen einer axialen Position sowie ggf. einer Drehung des Kupplungsschafts relativ zur Hülse um die Längsachse, so dass die korrespondierenden Haltestrukturen der Hülse und des Kupplungsschafts nur noch in radialer Richtung voneinander getrennt sind, wird durch manuelle Krafteinwirkung von außen oder durch elastische Rückstellkräfte der Querschnitt der Hülse und/oder des Kupplungsschafts in die jeweilige Arbeitsform überführt, so dass die Haltestrukturen miteinander zusammenwirken und der Kupplungsschaft in der Hülse gehalten ist. Somit ist der Instrumenteneinsatz mit dem Schaft verbunden. Ferner kann an einem proximalen Ende des Schafts ein Handgriff angesetzt und sowohl mit einem Schaftrohr als auch ggf. mit dem Übertragungselement verbunden werden.

In dem Fall, dass bei dem endoskopischen Instrument das Querschnittsprofil der Hülse und/oder des Kupplungsschafts zum Lösen des Kupplungsschafts aus der Hülse durch Temperatureinwirkung veränderbar ist, wird zum Bereitstellen des Instruments die Hülse auf eine Montagetemperatur erwärmt und sodann der Kupplungsschaft in die Hülse eingeführt. Nach Abkühlen der Hülse ist dann der Kupplungsschaft fest in der Hülse gehalten und somit der Instrumenteneinsatz mit dem Schaft verbunden. Zum Zerlegen des Instrumentes wird die Hülse wieder auf die Montagetemperatur erwärmt und dadurch geweitet, so dass der Kupplungsschaft aus der Hülse entnommen werden kann. Zum Bereitstellen bzw. zum Zerlegen des Instruments können weitere Schritte, wie oben erwähnt, vorgesehen sein.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Aspekte der Erfindung ergeben sich aus der nachfolgenden Beschreibung bevorzugter Ausführungsbeispiele und der beigefügten Zeichnung. Es zeigen:
Fig. 1 ein endoskopisches Instrument gemäß einer Ausführungsform der vorliegenden Erfindung in einer Seitenansicht;
Fig. 2 ein endoskopisches Instrument gemäß einer weiteren Ausführungsform der Erfindung in einer Teilansicht;
Fig. 3 den distalen Endbereich des Schafts des Instruments gemäß Fig. 2 mit von dem Schaft gelöstem Instrumenteneinsatz;
Fig. 4a bis 4c den distalen Endbereich des Schafts des Instruments gemäß Fig. 2 mit eingesetztem Instrumenteneinsatz bei geöffneter bzw. geschlossener Kupplung in unterschiedlichen Darstellungen;
Fig. 5 den distalen Endbereich des Schafts mit Instrumenteneinsatz gemäß Fig. 4b im Längsschnitt mit angedeuteten Querschnitten in schematischer Darstellung;
Fig. 6a und 6b zwei Varianten des Ausführungsbeispiels gemäß Fig. 2;
Fig. 7 die Wirkungsweise der Kupplung gemäß einem Ausführungsbeispiel der Erfindung in symbolischer Darstellung;
Fig. 8a bis 8c die Wirkungsweise der Kupplung gemäß einem weiteren Ausführungsbeispiel der Erfindung in schematischer Darstellung.

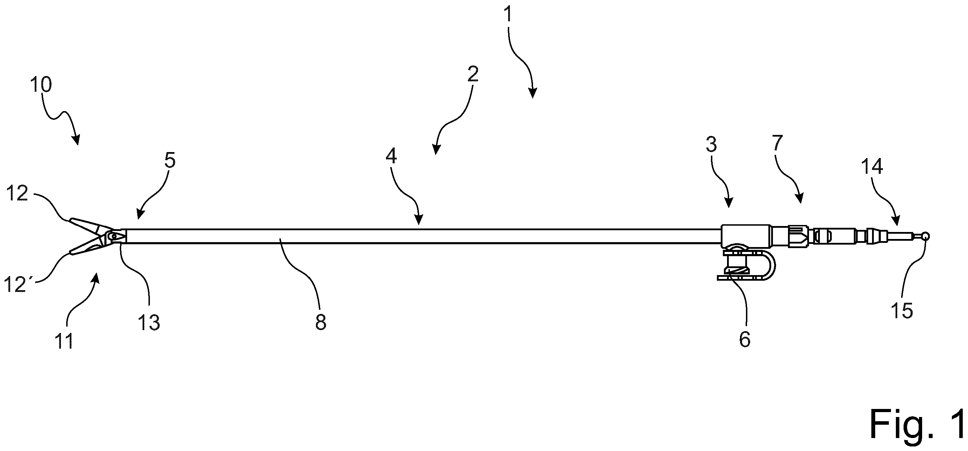

Wie in Fig. 1 dargestellt ist, umfasst ein medizinisches endoskopisches Instrument 1 gemäß einem Ausführungsbeispiel der vorliegenden Erfindung einen Schaft 2 und einen Instrumenteneinsatz 10. Der Schaft 2 umfasst einen proximalen Abschnitt 3, einen langerstreckten mittleren Abschnitt 4 und einen distalen Endabschnitt 5. Der Schaft 2 ist zur Einführung in einen körperinneren Hohlraum bemessen, beispielsweise zur Einführung in einen Arbeitskanal eines Endoskops, das bei einem endoskopischen Eingriff in den körperinneren Hohlraum eingeführt wird. Der proximale Abschnitt 3 des Schafts 2 kann beispielsweise einen Spülanschluss 6 aufweisen sowie einen Verbindungsmechanismus 7 zur Verbindung mit einem nicht dargestellten Handgriff. Mittels des Verbindungsmechanismus 7 kann der Handgriff drehbar oder drehfest mit dem Schaft 2 verbunden werden.

Am distalen Endabschnitt 5 des Schafts 2 ist ein Werkzeug 11 angeordnet, das im dargestellten Ausführungsbeispiel als Schere mit zwei relativ zum distalen Endabschnitt 5 des Schafts 2 schwenkbaren Scherenblättern 12, 12' ausgebildet ist. Das Werkzeug 11 ist Teil des Instrumenteneinsatzes 10, der distalseitig in den Schaft 2 eingesetzt ist. Der Instrumenteneinsatz 10 umfasst weiterhin eine Basis 13, die distalseitig als Gabel ausgebildet ist, worin die Scherenblätter 12, 12' schwenkbar gelagert sind. Die Basis 13 ist mit einem Kupplungsschaft in den distalen Endabschnitt 5 des Schafts 2 eingesetzt und mit diesem verbunden, wofür der distale Endabschnitt 5 als Hülse ausgebildet ist, wie unten beispielhaft erläutert wird.

Innerhalb des Schafts 2 ist eine Zugstange 14 in Längsrichtung des Schafts 10 verschiebbar angeordnet. Das proximale Ende der Zugstange 14 wird durch ein Verbindungselement gebildet, beispielsweise eine Kugel 15, die mit einem beweglichen Teil des nicht dargestellten Handgriffs verbunden werden kann, um durch Betätigen des beweglichen Teils des Handgriffs die Zugstange 14 in Längsrichtung zu verschieben. Die Zugstange 14 kann sowohl Zug- als auch Schubkräfte in Längsrichtung des Schafts 10 übertragen. Die Zugstange 14 ist gelenkig mit den Scherenblättern 12, 12' verbunden und bildet ebenfalls einen Teil des Instrumenteneinsatzes 10. Durch Verschieben der Zugstange 14 in proximaler oder distaler Richtung können die Scherenblätter 12, 12' geöffnet oder geschlossen werden. Der Schaft 2 ist als metallisches Schaftrohr ausgebildet, das von einer elektrisch isolierenden Umhüllung 8 umschlossen ist. Am Handgriff oder am Schaft 2 können beispielsweise elektrische Anschlüsse für HF-Spannung angeordnet sein.

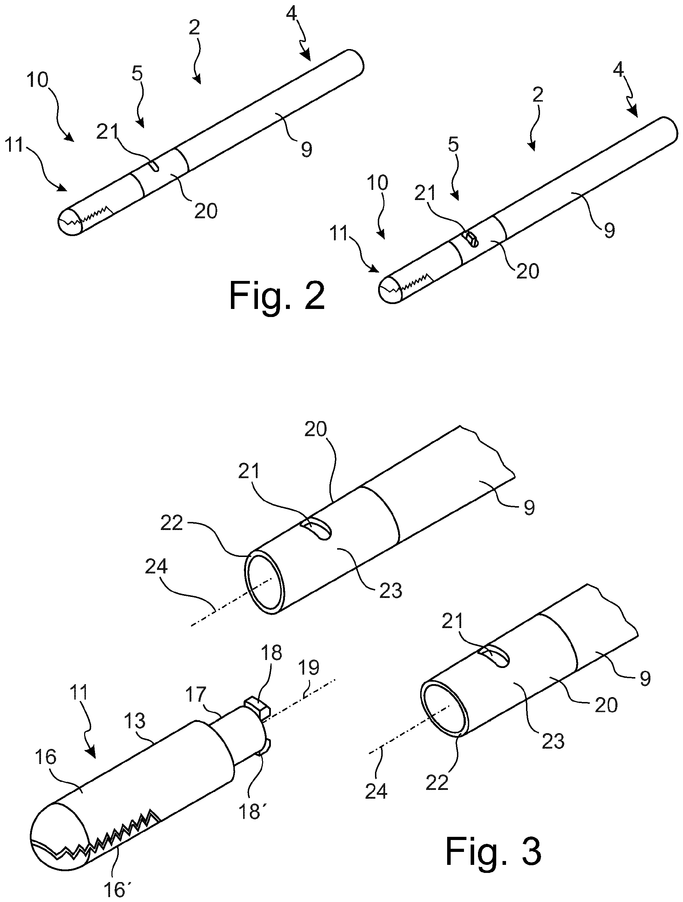

Fig. 2 zeigt einen Schaft 2 gemäß einem weiteren Ausführungsbeispiel der Erfindung in einer schräg aus distaler Richtung gesehenen Teilansicht. Wie in Fig. 2 angedeutet ist, wird der mittlere Abschnitt 4 des Schafts durch ein metallisches Schaftrohr 9 gebildet; die Umhüllung 8 (s. Fig. 1) ist nicht dargestellt. Der distale Endabschnitt 5 des Schafts 2 ist als Hülse 20 ausgebildet, die einen Teil einer Kupplung zur lösbaren Verbindung des Instrumenteneinsatzes 10 mit dem Schaft 2 bildet. Die Hülse 20 weist zwei, einander gegenüberliegend angeordnete, sich jeweils in Umfangsrichtung erstreckende Querschlitze 21, 21' auf, von denen in Fig. 2 und 3 nur der obere Querschlitz 21 sichtbar ist. Die Begriffe "oben" sowie "horizontal" und "vertikal" beziehen sich hier und im Folgenden auf die in den Figuren dargestellte Lage des Instruments, wobei bei einer Verwendung das endoskopische Instrument eine beliebige andere Lage einnehmen kann.

Die Hülse 20 ist zumindest im Bereich der Querschlitze 21, 21' elastisch verformbar, so dass die Hülse durch Krafteinwirkung von außen in radialer Richtung unterschiedliche Querschnittsformen annehmen kann. Im Bereich der Querschlitze 21, 21' weist die Hülse 20 somit ein elastisch verformbares Querschnittsprofil auf. In der linken Abbildung von Fig. 2 und 3 hat die Hülse 20 eine Montageform, während sie in der rechten Abbildung von Fig. 2 und 3 eine Arbeitsform hat. Die Hülse 20 ist proximalseitig fest mit dem Schaftrohr 9 verbunden oder kann einstückig mit dem Schaftrohr 9 ausgebildet sein, so dass eine Außenkontur des Schaftrohrs 9 glatt in die der Hülse 20 übergeht.

Im proximalen Abschnitt hat die Hülse 20 eine weitgehend unveränderliche, näherungsweise dem beispielsweise kreisförmigen oder ovalen Querschnitt des Schaftrohrs 9 entsprechende Querschnittform. Im Bereich der Querschlitze 21, 21' sowie distalseitig der Querschlitze 21, 21' ist die Hülse 20 jedoch elastisch verformbar und nimmt, wenn keine äußere Krafteinwirkung erfolgt, die Arbeitsform ein bzw. ist elastisch in die Arbeitsform vorgespannt. Wie in Fig. 3 an der distalen Endfläche 22 der Hülse 20 zu erkennen ist, ist im dargestellten Beispiel die Arbeitsform (rechte Abbildung der Hülse 20) kreisförmig oder breitoval, d.h. mit einer horizontal gerichteten Hauptachse. Durch Krafteinwirkung von außen in radialer Richtung auf einander in horizontaler Richtung gegenüberliegende Oberflächenbereiche, die in Umfangsrichtung um etwa 90° gegenüber der Mitte der Querschlitze 21, 21' versetzt sind und von denen nur einer in Fig. 2 und 3 sichtbar ist, welche Oberflächenbereiche hier als Druckpunkte 23, 23' bezeichnet werden, kann die Hülse 20 derart verformt werden, dass sie die Montageform einnimmt, die im dargestellten Beispiel hochoval ist, d.h. eine vertikal gerichtete Hauptachse hat (linke Abbildung der Hülse 20). Die Hülse 20 ist vorzugsweise aus metallischem Glas hergestellt, welches sowohl eine hohe Festigkeit als auch eine hohe Elastizität ermöglicht.

Der Instrumenteneinsatz 10 weist ein Werkzeug 11 auf, das im dargestellten Beispiel als Fasszange ausgebildet ist, die ein feststehendes Maulteil 16 umfasst, das mit der Basis 13 des Werkzeugs fest verbunden ist, und ein bewegliches Maulteil 16', das schwenkbar an der Basis 13 angelenkt ist. Ein proximaler Endbereich der Basis 13 ist als Kupplungsschaft 17 ausgebildet, an dessen proximalem Ende, in vertikaler Richtung einander gegenüberliegend, zwei Nocken 18, 18' angeordnet sind, die jeweils in radialer Richtung über die Oberfläche des Kupplungsschafts 17 hinaus ragen. Der Instrumenteneinsatz 10 umfasst ferner eine nicht dargestellte Zugstange, die durch den Kupplungsschaft 17 und die Basis 13 hindurchgeführt und mit dem beweglichen Maulteil 16' verbunden ist und die zum Einführen in den Schaft 2 des endoskopischen Instruments 1 bis zu dessen proximalem Ende bemessen ist.

Die Nocken 18, 18' sind korrespondierend zu den Querschlitzen 21, 21' der Hülse 20 ausgebildet, so dass die Nocken 18, 18' in die Querschlitze 21, 21' eingreifen können, wenn die Hülse 20 die Arbeitsform hat (rechte Abbildung in Fig. 2 und 3, wobei in der rechten Abbildung in Fig. 2 der in den Querschlitz 21 eingreifende Nocken 18 übertrieben dargestellt ist). Andererseits ist in der Montageform der Hülse 20 (linke Abbildung des Schafts 2 in Fig. 2 und 3) die Innenweite der Hülse 20 im Bereich der Querschlitze 21, 21' in vertikaler Richtung größer als der in vertikaler Richtung gemessene Durchmesser der durch die beiden Nocken 18, 18' gebildeten Eingriffsstruktur, d.h. der vertikale Abstand zwischen den Außenseiten der Nocken 18, 18'; in der Montageform können die Nocken 18, 18' daher nicht mehr beide zugleich in die Querschlitze 21, 21' eingreifen.

Wird die Hülse 20 durch seitlichen Druck auf die Druckpunkte 23, 23' in die Montageform gebracht und in dieser gehalten, so kann der Kupplungsschaft 17 in die Hülse 20 eingeführt werden, bis die Nocken 18, 18' in axialer Richtung auf Höhe der Querschlitze 21, 21' liegen. Sofern erforderlich, können die Nocken 18, 18' durch eine Drehung des Kupplungsschafts 17 um seine Längsachse in den Querschlitzen 21, 21' entsprechende Winkelpositionen gebracht werden. Die Querschlitze 21, 21' sind dann nur noch in radialer Richtung von den Nocken 18, 18' abgehoben. Wird sodann die seitliche Krafteinwirkung beendet, so greifen die Nocken 18, 18' in die Querschlitze 21, 21' ein. In diesem Zustand wird der Kupplungsschaft 17 und damit der Instrumenteneinsatz 10 fest am distalen Ende des Schafts 2 gehalten. Dabei kann durch die Ausgestaltung der Querschlitze 21, 21' und der Nocken 18, 18', ggf. zusätzlich im Zusammenwirken mit einem Anliegen der Basis 13 an der distalen Endfläche 22 der Hülse, ein axiales Spiel begrenzt oder vermieden werden, ebenso wie hierdurch ggf. eine drehfeste Verbindung bewirkt werden kann. Durch erneuten seitlichen Druck auf die Druckpunkte 23, 23' kann die Hülse 20 wieder in die Montageform überführt werden, so dass die Hülse 20 im Bereich der Querschlitze 21, 21' von den Nocken 18, 18' abgehoben wird und der Kupplungsschaft 17 aus der Hülse 20 in axialer Richtung herausgezogen werden kann.

Wie in Fig. 2 und 3 ersichtlich ist, fluchtet eine Längsachse 19 der Basis 13 und des Kupplungsschafts 17 im montierten Zustand mit einer Längsachse 24 des Schafts 2 und der Hülse 20, und das Werkzeug 11 ist in einer distalseitigen Verlängerung des Schafts 2 angeordnet.

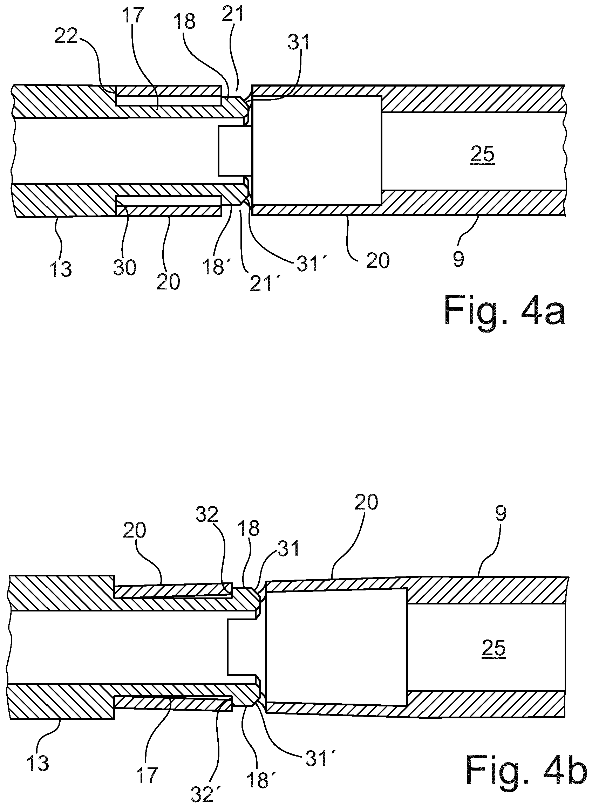

In Fig. 4a und 4b ist die durch den Kupplungsschaft 17 und die Hülse 20 gebildete Kupplung in offenem bzw. geschlossenem Zustand jeweils in einem vertikalen Längsschnitt gezeigt. In Fig. 4a ist dargestellt, dass der Kupplungsschaft 17 in die Hülse 20 eingeführt ist. Bei der axial gerichteten Einführbewegung wird die Hülse 20 über proximalseitige Anlaufschrägen 31, 31' der Nocken 18, 18' in vertikaler Richtung aufgeweitet. Hierdurch wird das Einführen des Kupplungsschafts 17 in die Hülse 20 erleichtert; zusätzlich kann zum Erreichen und Halten der Montageform eine seitliche Druckkraft auf die Druckpunkte 23, 23' ausgeübt werden (s.o.). In dem in Fig. 4a gezeigten Zustand ist der Kupplungsschaft 17 in axialer Richtung so weit in die Hülse 20 eingeführt, dass eine Stufe 30 der Basis 13 an der distalen Endfläche 22 der Hülse 20 anliegt. In dieser Position sind die Nocken 18, 18' radial innerhalb der Querschlitze 21, 21' angeordnet. In Fig. 4a ist die Hülse 20 in der Montageform dargestellt, in der die Nocken 18, 18' noch nicht in die Querschlitze 21, 21' eingreifen.

In Fig. 4b ist die Hülse 20 in der Arbeitsform gezeigt, die die Hülse 20 nach Erreichen der in Fig. 4a gezeigten Position durch eine elastische Rückstellkraft und ggf. durch Wegfall der seitlichen Druckkraft einnimmt. In der Arbeitsform greifen die Nocken 18, 18' in die Querschlitze 21, 21' ein, und die Hülse 20 greift mit den distalseitig an die Querschlitze 21, 21' anschließenden Bereichen in den durch eine distalseitige Stufe 32, 32' der Nocken 18, 18' gebildeten Hinterschnitt ein. Hierdurch wird der Kupplungsschaft 17 und damit die Basis 13 des Instrumenteneinsatzes 10 fest am Schaft 2 gehalten. Insbesondere ist durch die als Anschlag wirkende Stufe 30 der Basis 13 und die distale Seite der Nocken 18, 18' eine axiale Position der Basis 13 relativ zum Schaft 2 festgelegt. Ferner kann durch die Erstreckung der Nocken 18, 18' in Umfangsrichtung im Zusammenwirken mit einer entsprechenden Erstreckung der Querschlitze 21, 21' eine Drehposition der Basis 13 relativ zum Schaft 2 festgelegt sein.

Wie in Fig. 4b ebenfalls erkennbar ist, liegt die Hülse 20 mit einem Eingriffsabschnitt, insbesondere am distalen Ende, in vertikaler Richtung an dem Kupplungsschaft 17 an. Hierdurch kann eine gegen entsprechende Biegebelastungen steife Verbindung der Basis 13 mit dem Schaft 2 erreicht werden. Die Basis 13 mit dem Kupplungsschaft 17 sowie die Hülse 20 und das Schaftrohr 9 bilden einen in Längsrichtung durchgehenden Innenraum 25, durch den die nicht dargestellte Zugstange, mit der das Werkzeug 11 betätigt werden kann, hindurchgeführt ist. Das Schaftrohr 9 hat eine größere Wandstärke als die Hülse 20, jedoch eine Außenkontur, die glatt in die Außenkontur der Hülse übergeht. Die Hülse 20 kann einstückig mit dem Schaftrohr 9 ausgeführt sein.

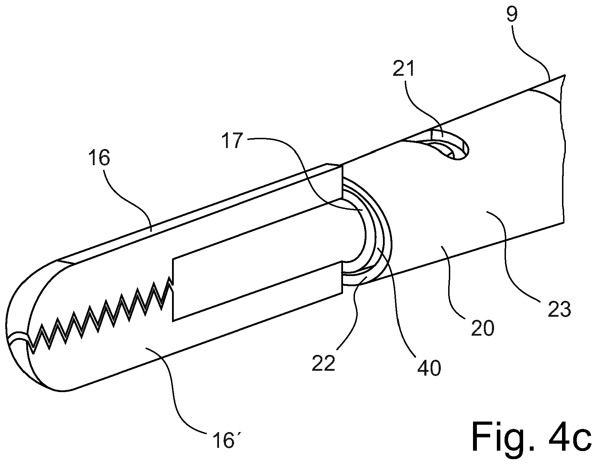

In Fig. 4c ist die Kupplung in geschlossenem Zustand in einer teilweise geschnittenen Schrägansicht gezeigt. Wie in Fig. 4c zu erkennen ist, ist im Bereich des Druckpunkts 23 zwischen einer äußeren Oberfläche des Kupplungsschafts 17 und einer inneren Oberfläche der Hülse 20 ein Hohlraum 40 vorhanden, um die Hülse 20 ausreichend zusammendrücken zu können, so dass die Nocken 18, 18' aus den Querschlitzen 21, 21' gelöst werden können.

Ein entsprechender Hohlraum ist auch auf der in Fig. 4c nicht sichtbaren gegenüberliegenden Seite des Querschnitts vorhanden. Gegen eine Biegebelastung in horizontaler Richtung ist die Kupplung bei diesem Ausführungsbeispiel dadurch steif ausgebildet, dass die distale Endfläche 22 der Hülse an der Stufe 30 der Basis 13 anliegt und durch die distalseitige Stufe 32, 32' der Nocken 18, 18', die jeweils an der distalen Seite des betreffenden Querschlitzes 21, 21' anliegt, gehalten wird; hierfür sind die betreffenden Abschnitte der Hülse 20 und des Kupplungsschafts 17 bezüglich ihrer Länge mit enger Toleranz aufeinander abgestimmt. Die Zugstange und der Mechanismus zum Schwenken des beweglichen Maulteils 16' sind in Fig. 4c nicht dargestellt.

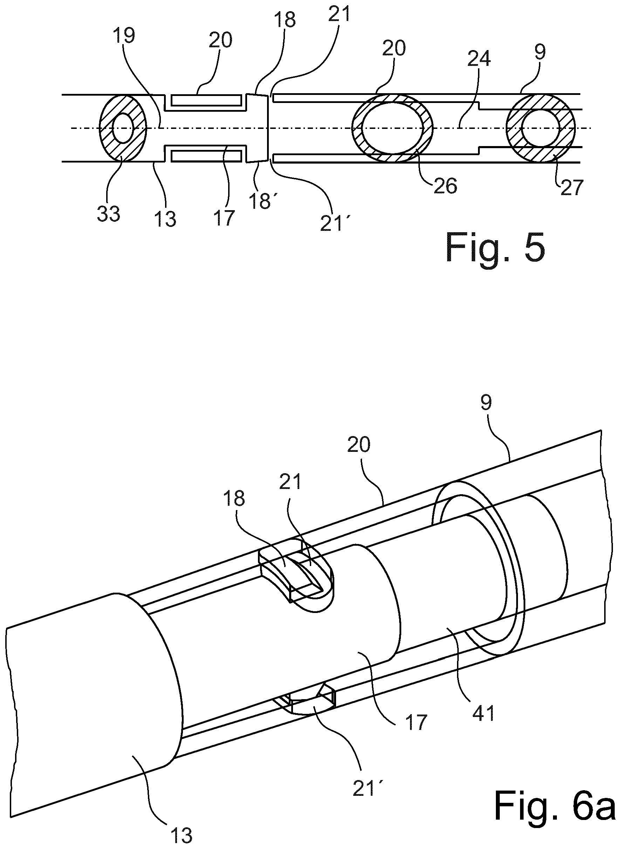

In Fig. 5 ist in einer vereinfachten schematischen Darstellung die Kupplung im geschlossenen Zustand, d.h. entsprechend Fig. 4b, ebenfalls in einem vertikalen Längsschnitt gezeigt, wobei zur Verdeutlichung mehrere Querschnitte angedeutet sind. Im geschlossenen Zustand der Kupplung, in dem die Querschnittsform der Hülse 20 die Arbeitsform ist, greift die Hülse 20 mit ihren distalseitig an die Querschlitze 21, 21' anschließenden Bereichen in den durch die distalseitige Stufe 32, 32' der Nocken 18, 18' gebildeten Hinterschnitt ein, und die Nocken 18, 18' greifen in die Querschlitze 21, 21' ein (s. Fig. 4a und 4b). Wie in Fig. 5 symbolisch angedeutet ist, hat die Hülse 20 im Bereich der Querschlitze 21, 21' in der Arbeitsform einen breitovalen Querschnitt 26. Das Schaftrohr 9, das sich proximalseitig an die Hülse 20 anschließt, hat einen kreisrunden Querschnitt 27. Die Basis 13 hat ebenfalls einen kreisförmigen Querschnitt 33.

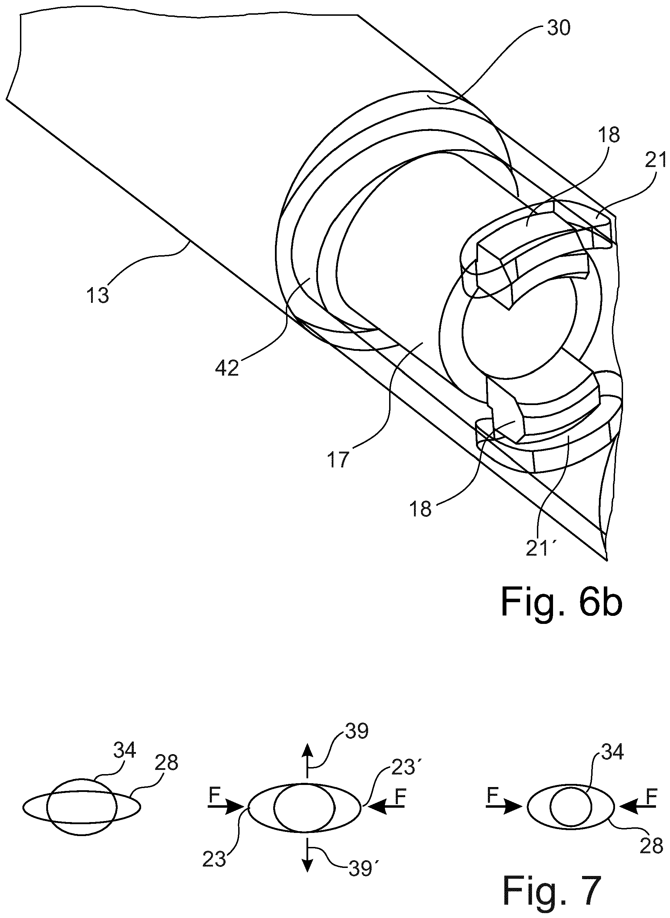

In Fig. 6a und 6b sind zwei Varianten des zuvor beschriebenen Ausführungsbeispiels jeweils in teiltransparenter Ansicht dargestellt. Bei der in Fig. 6a gezeigten Variante ist der Kupplungsschaft 17 in proximaler Richtung verlängert, so dass dieser im geschlossenen Zustand der Kupplung durch die Hülse 20 hindurch bis in den Schaft 9 hinein ragt. Die proximalseitige Verlängerung 41 des Kupplungsschafts 17 liegt eng toleriert bzw. spielfrei an der Innenseite des Schafts 9 an. Hierdurch kann die Kupplung zusätzlich versteift werden, insbesondere auch in horizontaler Richtung.

Bei der in Fig. 6b dargestellten Variante weist der Kupplungsschaft 17 distalseitig der Stufe 30 der Basis 13 einen Stützabschnitt 42 auf, der ein Querschnittsprofil hat, das an die Innenkontur der Hülse 20 in der Arbeitsform angepasst ist und spielfrei an dieser anliegt. Der Stützabschnitt 42 weist nur eine kurze Längserstreckung auf, um eine Verformung der Hülse 20 zum Lösen der Nocken 18, 18' aus den Querschlitzen 21, 21' nicht zu behindern. Auch hierdurch kann eine erhöhte Steifigkeit auch in horizontaler Richtung erreicht werden, wobei das Betätigungsprofil 42 eine zusätzliche Verdrehsicherung zwischen dem Schaft 9 und der Basis 13 darstellen kann.

In Fig. 7 ist das Öffnen der Kupplung symbolisch mittels der Querschnittskonturen dargestellt. Im geschlossenen Zustand (linke Abbildung) ist die Querschnittskontur 28 der Hülse 20 im Bereich einer Haltestruktur derart abgeflacht, dass diese in einen Hinterschnitt des Kupplungsschafts 17, welcher mit kreisförmiger Querschnittskontur 34 dargestellt ist, eingreift. Durch beidseitigen Druck (Kraft F) auf die Druckpunkte 23, 23', die den Scheitelpunkten der breitovalen Querschnittsform entsprechen, kann das Querschnittsprofil in den dazwischenliegenden Umfangsbereichen in Richtung der Pfeile 39, 39' gespreizt und somit die Abflachung des Querschnitts verringert werden (mittlere Abbildung), bis schließlich die Haltestruktur der Hülse aus dem Hinterschnitt des Kupplungsschafts 17 ausgehoben wird und die Kupplung geöffnet ist (rechte Abbildung).

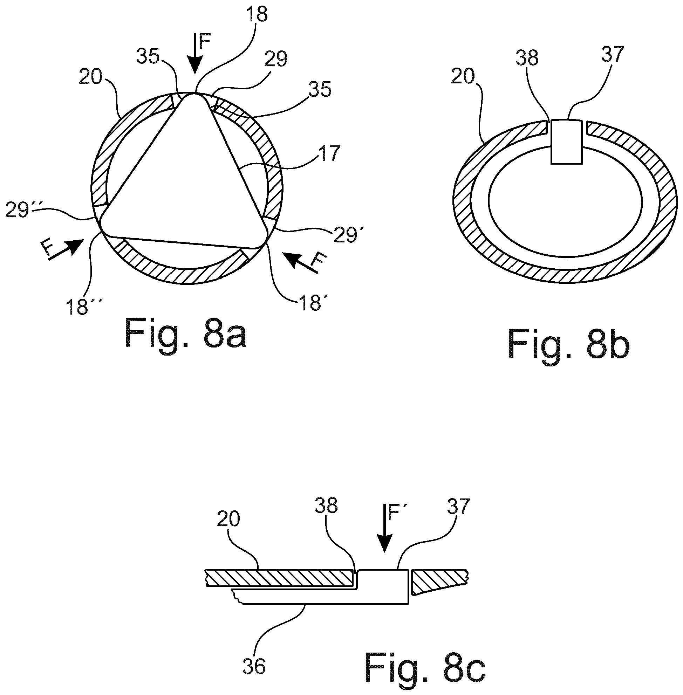

Bei einem weiteren Ausführungsbeispiel , welches in Fig. 8a bis 8c schematisch dargestellt ist, weist der Kupplungsschaft 17 einen Querschnitt auf, der näherungsweise die Form eines gleichseitigen Dreiecks hat, wobei die Ecken des Dreiecks als Nocken 18, 18', 18" ausgebildet sind, die in der Arbeitsform jeweils in Durchbrüche 29, 29', 29" der Hülse 20, die in Umfangsrichtung zueinander um jeweils 120° versetzt sind, eingreifen. Der Kupplungsschaft 17 hat ein derart verformbares Querschnittsprofil, dass die Nocken 18, 18', 18" nach innen gedrückt werden können. Die Kupplung kann dadurch gelöst werden, dass jeweils in Richtung der Pfeile manuell eine Kraft F auf die Nocken 18, 18', 18" ausgeübt wird, so dass der Kupplungsschaft 17 in die Montageform überführt wird, in der die Nocken 18, 18', 18 "nicht mehr in die Durchbrüche 29, 29', 29" eingreifen und der Kupplungsschaft 17 aus der Hülse 20 in axialer Richtung herausgezogen werden kann (nicht dargestellt). Hier, wie bei den zuvor beschriebenen Ausführungsbeispielen, kann eine Kraft F im Bereich von einem oder wenigen N, beispielsweise ca. 5 N, hierfür ausreichend sein.

Bei dem in Fig. 8a gezeigten Ausführungsbeispiel weisen die Nocken 18, 18', 18" jeweils beidseitig in Umfangsrichtung Anlaufschrägen 35 auf. Hierdurch ergibt sich die Möglichkeit, alternativ oder zusätzlich zur manuellen Ausübung der seitlichen Druckkraft die Hülse 20 relativ zum Kupplungsschaft 17 um die Längsachse zu verdrehen, wodurch die Nocken 18, 18', 18" über die jeweiligen Anlaufschrägen 35 in radialer Richtung eingedrückt werden und dadurch ebenfalls außer Eingriff mit den Durchbrüchen 29, 29', 29" gelangen. Auch hierdurch kann die Kupplung gelöst werden.

Ferner kann es alternativ oder zusätzlich vorgesehen sein, dass die Hülse 20 ein verformbares Querschnittsprofil aufweist. In diesem Fall kann durch seitliche Kraftausübung auf Druckpunkte, die in Umfangsrichtung zwischen den Durchbrüchen 29, 29', 29" liegen, ebenso wie durch die Rotation der Hülse 20 relativ zum Kupplungsschaft 17 die Hülse 20 verformt werden, so dass die Nocken 18, 18', 18" aus den Durchbrüchen 29, 29', 29" gelöst werden (nicht dargestellt).

Gemäß einer Variante der in Fig. 8a gezeigten Ausführungsform weist die Basis 13 bzw. der Kupplungsschaft 17 ferner eine federnde Lasche 36 auf, die eine Rastnase 37 trägt, welche bei geschlossener Kupplung in einen Durchbruch 38 der Hülse 20 eingreift und die in Umfangsrichtung keine Anlaufschrägen aufweist (s. Fig. 8b, 8c). Diese dient daher als Verdrehsicherung, um ein versehentliches Lösen der Kupplung durch ein bei der Verwendung des Instruments auftretendes Drehmoment zu verhindern. Zum Lösen der Kupplung muss daher bei dieser Variante die Rastnase 37 durch Ausübung einer radialen Kraft F' eingedrückt werden, um sodann den Kupplungsschaft 17 um die Längsachse relativ zur Hülse 20 zu verdrehen.

Der Übersichtlichkeit halber sind nicht in allen Figuren alle Bezugszeichen dargestellt. Zu einer Figur nicht erläuterte Bezugszeichen haben die gleiche oder eine entsprechende Bedeutung wie in den übrigen Figuren.

### Bezugszeichenliste

- 1: Instrument
- 2: Schaft
- 3: Proximaler Abschnitt
- 4: Mittlerer Abschnitt
- 5: Distaler Endabschnitt
- 6: Spülanschluss
- 7: Verbindungsmechanismus
- 8: Umhüllung
- 9: Schaftrohr
- 10: Instrumenteneinsatz
- 11: Werkzeug
- 12, 12': Scherenblatt
- 13: Basis
- 14: Zugstange
- 15: Kugel
- 16, 16': Maulteil
- 17: Kupplungsschaft
- 18, 18', 18": Nocken
- 19: Längsachse
- 20: Hülse
- 21, 21': Querschlitz
- 22: Endfläche
- 23, 23`: Druckpunkt
- 24: Längsachse
- 25: Innenraum
- 26: Querschnitt
- 27: Querschnitt
- 28: Querschnittskontur
- 29, 29', 29": Durchbruch
- 30: Stufe
- 31, 31': Anlaufschräge
- 32, 32': Stufe
- 33: Querschnitt
- 34: Querschnittskontur
- 35: Anlaufschräge
- 36: Lasche
- 37: Rastnase
- 38: Durchbruch
- 39, 39': Pfeil
- 40: Hohlraum
- 41: Verlängerung
- 42: Stützabschnitt
- F, F': Kraft

## Patentansprüche

1. Endoskopisches Instrument (1) mit einem langerstreckten Schaft (2) und einem Instrumenteneinsatz (10), der mit einem distalen Endabschnitt (5) des Schafts (2) lösbar verbunden ist, wobei der distale Endabschnitt (5) des Schafts (2) als Hülse (20) und ein proximaler Endbereich einer Basis (13) des Instrumenteneinsatzes (10) als Kupplungsschaft (17) ausgebildet ist oder der distale Endabschnitt (5) des Schafts (2) als Kupplungsschaft (17) und ein proximaler Endbereich einer Basis (13) des Instrumenteneinsatzes (10) als Hülse (20) ausgebildet ist, wobei der Kupplungsschaft (17) lösbar in der Hülse (20) gehalten ist, wobei die Hülse (20) und/oder der Kupplungsschaft (17) zumindest abschnittsweise ein Querschnittsprofil aufweist, das zum Lösen des Kupplungsschafts (17) aus der Hülse (20) reversibel veränderbar ist, wobei die Hülse (20) eine erste Haltestruktur und der Kupplungsschaft (17) eine zweite Haltestruktur aufweist, wobei die Hülse (20) und/oder der Kupplungsschaft (17) durch reversible Verformung aus einer j eweiligen Arbeitsform, in der die erste Haltestruktur mit der zweiten Haltestruktur zum Halten des Kupplungsschafts (17) in der Hülse (20) angeordnet ist, in eine jeweilige Montageform, in der die erste Haltestruktur und die zweite Haltestruktur voneinander getrennt angeordnet sind, überführbar ist oder sind, wobei sich die erste und/oder die zweite Haltestruktur in Umfangsrichtung über einen oder mehrere Teil-Winkelbereiche der Hülse (20) bzw. des Kupplungsschafts (17) erstreckt , **dadurch gekennzeichnet, dass** die Hülse (20) mindestens einen außerhalb der Teil-Winkelbereiche gelegenen Druckpunkt (23, 23') zum Verformen des Querschnittsprofils der Hülse (20) aufweist.

2. Endoskopisches Instrument (1) nach Anspruch 1, **dadurch gekennzeichnet, dass** die Arbeitsform der Hülse (20) eine abgeflachte Form ist, wobei in den Scheitelpunkten der abgeflachten Form jeweils ein Druckpunkt (23, 23') angeordnet ist, und/oder die Montageform der Hülse (20) eine abgeflachte Form ist, wobei in Umfangsrichtung zwischen den Scheitelpunkten jeweils ein Druckpunkt (23, 23') angeordnet ist.

3. Endoskopisches Instrument (1) nach Anspruch 1, **dadurch gekennzeichnet, dass** das Querschnittsprofil zum Lösen des Kupplungsschafts (17) aus der Hülse (20) durch elastische Verformung der Hülse (20) bzw. des Kupplungsschafts (17) reversibel veränderbar ist.

4. Endoskopisches Instrument (1) nach Anspruch 1 oder 2, **dadurch gekennzeichnet, dass** das Querschnittsprofil ein im Wesentlichen geschlossenes Querschnittsprofil ist.

5. Endoskopisches Instrument (1) nach einem der vorhergehenden Ansprüche, **dadurch gekennzeichnet, dass** die Hülse (20) drei Druckpunkte (23, 23') aufweist, die jeweils um etwa 120° gegeneinander versetzt sind.

6. Endoskopisches Instrument (1) nach einem der vorherigen Ansprüche, **dadurch gekennzeichnet, dass** der Kupplungsschaft (17) einen Stützabschnitt (42) zur Abstützung eines Endabschnitts der Hülse (20) in der Arbeitsform aufweist.

7. Endoskopisches Instrument (1) nach einem der vorherigen Ansprüche, **dadurch gekennzeichnet, dass** die erste Haltestruktur eine Eingriffsstruktur und die zweite Haltestruktur ein zum Eingreifen in die erste Haltestruktur ausgebildetes Eingriffselement ist oder umgekehrt.

8. Endoskopisches Instrument (1) nach einem der vorherigen Ansprüche, **dadurch gekennzeichnet, dass** die zweite Haltestruktur einen radial vorstehenden Nocken (18, 18', 18") aufweist und die erste Haltestruktur einen korrespondierenden Durchbruch (29, 29', 29") aufweist, in den der Nocken (18, 18', 18") eingreift.

9. Endoskopisches Instrument (1) nach einem der vorherigen Ansprüche, **dadurch gekennzeichnet, dass** die erste und die zweite Haltestruktur zum drehfesten Halten des Kupplungsschafts (17) in der Hülse (20) zusammenwirken.

10. Endoskopisches Instrument (1) nach einem der vorherigen Ansprüche, **dadurch gekennzeichnet, dass** die erste und/oder die zweite Haltestruktur in Umfangsrichtung ein- oder beidseitig eine Anlaufschräge (35) aufweist bzw. aufweisen.

11. Endoskopisches Instrument (1) nach einem der vorhergehenden Ansprüche, **dadurch gekennzeichnet, dass** der Kupplungsschaft (17) eine federnde Lasche (36) mit einer in einen Durchbruch (29) der Hülse (20) eingreifenden Rastnase (37) aufweist.

12. Endoskopisches Instrument (1) nach einem der vorhergehenden Ansprüche, **dadurch gekennzeichnet, dass** erste und/oder die zweite Haltestruktur in axialer Richtung in einer Einführrichtung eine Anlaufschräge (31, 31') aufweist.

13. Endoskopisches Instrument (1) nach einem der vorhergehenden Ansprüche, **dadurch gekennzeichnet, dass** die erste und die zweite Haltestruktur miteinander zusammenwirkende Reibflächen umfassen und/oder für eine Mikroverzahnung ausgebildet sind.

14. Endoskopisches Instrument (1) nach einem der vorhergehenden Ansprüche, **dadurch gekennzeichnet, dass** die Hülse (20) und/oder der Kupplungsschaft (17) zumindest teilweise aus metallischem Glas besteht.

15. Endoskopisches Instrument (1) nach einem der vorhergehenden Ansprüche, **dadurch gekennzeichnet, dass** das Querschnittsprofil zum Lösen des Kupplungsschafts (17) aus der Hülse (20) durch Temperatureinwirkung veränderbar ist.

## Claims

1. Endoscopic instrument (1) comprising an elongate shaft (2) and an instrument insert (10) which is releasably connected to a distal end portion (5) of the shaft (2), the distal end portion (5) of the shaft (2) being designed as a sleeve (20) and a proximal end region of a base (13) of the instrument insert (10) being designed as a coupling shaft (17) or the distal end portion (5) of the shaft (2) being designed as a coupling shaft (17) and a proximal end region of a base (13) of the instrument insert (10) being designed as a sleeve (20), the coupling shaft (17) being releasably held in the sleeve (20), the sleeve (20) and/or the coupling shaft (17) having, at least in portions, a cross-sectional profile which can be reversibly changed in order to release the coupling shaft (17) from the sleeve (20), the sleeve (20) having a first holding structure and the coupling shaft (17) having a second holding structure, the sleeve (20) and/or the coupling shaft (17) being able to be converted by reversible deformation from a relevant working shape, in which the first holding structure is arranged together with the second holding structure in order to hold the coupling shaft (17) in the sleeve (20), into a relevant assembly shape, in which the first holding structure and the second holding structure are arranged separately from one another, the first and/or the second holding structure extending in the circumferential direction over one or more partial angular regions of the sleeve (20) or the coupling shaft (17), **characterized in that** the sleeve (20) has at least one pressure point (23, 23') for deforming the cross-sectional profile of the sleeve (20), which pressure point is located outside the partial angular regions.

2. Endoscopic instrument (1) according to claim 1, **characterized in that** the working shape of the sleeve (20) is a flattened shape, a pressure point (23, 23') being arranged in each of the apices of the flattened shape, and/or the assembly shape of the sleeve (20) being a flattened shape, a pressure point (23, 23') being arranged in the circumferential direction between each of the apices.

3. Endoscopic instrument (1) according to claim 1, **characterized in that** the cross-sectional profile for releasing the coupling shaft (17) from the sleeve (20) can be reversibly changed by elastic deformation of the sleeve (20) or the coupling shaft (17).

4. Endoscopic instrument (1) according to either claim 1 or claim 2, **characterized in that** the cross-sectional profile is a substantially closed cross-sectional profile.

5. Endoscopic instrument (1) according to any of the preceding claims, **characterized in that** the sleeve (20) has three pressure points (23, 23'), each offset from each other by approximately 120°.

6. Endoscopic instrument (1) according to any of the preceding claims, **characterized in that** the coupling shaft (17) has a support portion (42) for supporting an end portion of the sleeve (20) in the working shape.

7. Endoscopic instrument (1) according to any of the preceding claims, **characterized in that** the first holding structure is an engagement structure and the second holding structure is an engagement element designed to engage in the first holding structure, or vice versa.

8. Endoscopic instrument (1) according to any of the preceding claims, **characterized in that** the second holding structure has a radially projecting cam (18, 18', 18") and the first holding structure has a corresponding opening (29, 29', 29") in which the cam (18, 18', 18") engages.

9. Endoscopic instrument (1) according to any of the preceding claims, **characterized in that** the first and second holding structures cooperate in order to rotationally fixedly hold the coupling shaft (17) in the sleeve (20).

10. Endoscopic instrument (1) according to any of the preceding claims, **characterized in that** the first and/or the second holding structure have/has a lifting slope (35) on one or both sides in the circumferential direction.

11. Endoscopic instrument (1) according to any of the preceding claims, **characterized in that** the coupling shaft (17) has a resilient tab (36) with a locking lug (37) engaging in an opening (29) in the sleeve (20).

12. Endoscopic instrument (1) according to any of the preceding claims, **characterized in that** the first and/or the second holding structure has a lifting slope (31, 31') in the axial direction in an insertion direction.

13. Endoscopic instrument (1) according to any of the preceding claims, **characterized in that** the first and the second holding structure comprise interacting friction surfaces and/or are designed for micro-toothing.

14. Endoscopic instrument (1) according to any of the preceding claims, **characterized in that** the sleeve (20) and/or the coupling shaft (17) consist(s) at least partially of metallic glass.

15. Endoscopic instrument (1) according to any of the preceding claims, **characterized in that** the cross-sectional profile can be changed by the influence of temperature in order to release the coupling shaft (17) from the sleeve (20).

## Revendications

1. Instrument endoscopique (1) comportant une tige (2) allongée et un insert d'instrument (10) qui est relié de manière amovible à une section d'extrémité distale (5) de la tige (2), dans lequel la section d'extrémité distale (5) de la tige (2) est réalisée sous forme de douille (20) et une zone d'extrémité proximale d'une base (13) de l'insert d'instrument (10) est réalisée sous forme de tige d'accouplement (17) ou la section d'extrémité distale (5) de la tige (2) est réalisée sous forme de tige d'accouplement (17) et une zone d'extrémité proximale d'une base (13) de l'insert d'instrument (10) est réalisée sous forme de douille (20), dans lequel la tige d'accouplement (17) est maintenue de manière amovible dans la douille (20), dans lequel la douille (20) et/ou la tige d'accouplement (17) présentent au moins dans certaines sections un profil de section transversale qui peut être modifié de manière réversible pour permettre de détacher la tige d'accouplement (17) de la douille (20), dans lequel la douille (20) présente une première structure de maintien et la tige d'accouplement (17) présente une seconde structure de maintien, dans lequel la douille (20) et/ou la tige d'accouplement (17) peuvent être transférées par déformation réversible d'une forme de travail respective, dans laquelle la première structure de maintien est disposée avec la seconde structure de maintien pour le maintien de la tige d'accouplement (17) dans la douille (20), vers une forme de montage respective dans laquelle la première structure de maintien et la seconde structure de maintien sont disposées séparément l'une de l'autre, dans lequel la première et/ou la seconde structure de maintien s'étendent dans la direction circonférentielle sur une ou plusieurs zones angulaires partielles de la douille (20) ou de la tige d'accouplement (17), **caractérisé en ce que** la douille (20) présente au moins un point de poussée (23, 23') situé à l'extérieur des zones angulaires partielles pour la déformation du profil de section transversale de la douille (20).

2. Instrument endoscopique (1) selon la revendication 1, **caractérisé en ce que** la forme de travail de la douille (20) est une forme aplatie, dans lequel respectivement un point de poussée (23, 23') est disposé dans les sommets de la forme aplatie, et/ou la forme de montage de la douille (20) est une forme aplatie, dans lequel respectivement un point de poussée (23, 23') est disposé dans la direction circonférentielle entre les sommets.

3. Instrument endoscopique (1) selon la revendication 1, **caractérisé en ce que** le profil de section transversale pour le détachement de la tige d'accouplement (17) de la douille (20) peut être modifié de manière réversible par déformation élastique de la douille (20) ou de la tige d'accouplement (17).

4. Instrument endoscopique (1) selon la revendication 1 ou 2, **caractérisé en ce que** le profil de section transversale est un profil de section transversale sensiblement fermé.

5. Instrument endoscopique (1) selon l'une des revendications précédentes, **caractérisé en ce que** la douille (20) présente trois points de poussée (23, 23') respectivement décalés d'environ 120° les uns par rapport aux autres.

6. Instrument endoscopique (1) selon l'une des revendications précédentes, **caractérisé en ce que** la tige d'accouplement (17) présente une section de support (42) permettant le support d'une section d'extrémité de la douille (20) dans la forme de travail.

7. Instrument endoscopique (1) selon l'une des revendications précédentes, **caractérisé en ce que** la première structure de maintien est une structure de mise en prise et la seconde structure de maintien est un élément de mise en prise réalisé pour venir en prise dans la première structure de maintien ou inversement.

8. Instrument endoscopique (1) selon l'une des revendications précédentes, **caractérisé en ce que** la seconde structure de maintien présente une came (18, 18', 18") faisant saillie radialement et la première structure de maintien présente un passage (29, 29', 29") correspondant dans lequel la came (18, 18', 18") vient en prise.

9. Instrument endoscopique (1) selon l'une des revendications précédentes, **caractérisé en ce que** la première et la seconde structure de maintien coopèrent pour maintenir de manière solidaire en rotation la tige d'accouplement (17) dans la douille (20).

10. Instrument endoscopique (1) selon l'une des revendications précédentes, **caractérisé en ce que** la première et/ou la seconde structure de maintien présentent un biseau d'attaque (35) d'un côté ou des deux côtés dans la direction circonférentielle.

11. Instrument endoscopique (1) selon l'une des revendications précédentes, **caractérisé en ce que** la tige d'accouplement (17) présente une patte élastique (36) comportant un ergot d'encliquetage (37) venant en prise dans un passage (29) de la douille (20).

12. Instrument endoscopique (1) selon l'une des revendications précédentes, **caractérisé en ce que** la première et/ou la seconde structure de maintien présentent un biseau d'attaque (31, 31') dans la direction axiale dans une direction d'introduction.

13. Instrument endoscopique (1) selon l'une des revendications précédentes, **caractérisé en ce que** la première et la seconde structure de maintien comprennent des surfaces de friction coopérant entre elles et/ou sont réalisées pour une microdenture.

14. Instrument endoscopique (1) selon l'une des revendications précédentes, **caractérisé en ce que** la douille (20) et/ou la tige d'accouplement (17) sont composées au moins partiellement de verre métallique.

15. Instrument endoscopique (1) selon l'une des revendications précédentes, **caractérisé en ce que** le profil de section transversale pour le détachement de la tige d'accouplement (17) de la douille (20) peut être modifié par action thermique.
